# EUROPEAN PATENT SPECIFICATION

(11) **EP 4 461 210 B1**
(45) Date of publication and mention of the grant of the patent: **24.06.2026**
(21) Application number: 23781188.0
(22) Date of filing: 08.03.2023
(51) Int. Cl.: A61B 5/0205, A61B 5/00, A61B 5/024, A61B 5/332, A61B 5/0537, A61B 5/1455

(54) **ELECTRONIC APPARATUS INCLUDING PLURALITY OF ELECTRODE CONNECTION STRUCTURES**
ELEKTRONISCHE VORRICHTUNG MIT MEHREREN ELEKTRODENVERBINDUNGSSTRUKTUREN
APPAREIL ÉLECTRONIQUE COMPRENANT UNE PLURALITÉ DE STRUCTURES DE CONNEXION D'ÉLECTRODES

(30) Priority: 29.03.2022 KR 20220038499; 25.04.2022 KR 20220050605
(43) Date of publication of application: 13.11.2024
(73) Proprietor: SAMSUNG ELECTRONICS CO., LTD., Yeongtong-gu Suwon-si, Gyeonggi-do 16677 (KR)
(72) Inventor: JANG, Donghoo, Suwon-si Gyeonggi-do 16677 (KR); KANG, Junghyun, Suwon-si Gyeonggi-do 16677 (KR); JUNG, Jonggwan, Suwon-si Gyeonggi-do 16677 (KR); JEONG, Changhoon, Suwon-si Gyeonggi-do 16677 (KR)
(74) Representative: Gulde & Partner
(86) International application number: PCT/KR2023/003184
(87) International publication number: WO 2023/191330

(56) References cited:
- WO-A1-2021/075748
- WO-A1-2021/137492
- CN-A- 104 997 503
- CN-A- 110 794 666
- GB-A- 2 592 205
- KR-A- 20160 044 811
- KR-A- 20200 100 487
- KR-A- 20210 015 250
- US-A1- 2014 125 491
- US-B1- 10 485 478

## Description

### BACKGROUND

### Field

The disclosure relates to an electronic device including a plurality of electrical connection structures for electrodes of a biosensor module.

### Description of Related Art

With the common use of portable devices such as smartphones, wearable electronic devices (e.g., smart watches) used in conjunction with the smartphones have been increasingly used. The wearable electronic devices may be connected with the smartphones through wired or wireless communication and may provide various functions or operations provided by the smartphones to users. Due to the convenience, the spread of wearable electronic devices such as smart watches has been on the rise.

An electronic device that can be worn on a human body may remain in contact with a user's body for a considerable amount of time and may thus be usefully used in medical or health care. For example, the electronic device may detect the user's biometric information, such as photoplethysmogram (PPG), sleep section, skin temperature, heart rate, electrocardiogram, or body composition, depending on a sensor mounted therein, and the detected biometric information may be used for health care of the user.

A wearable electronic device may have a limitation in the size of the set due to the nature of the device. To provide various functions through the wearable electronic device, various electronic parts may be disposed inside the electronic device. In the design of the wearable electronic device having a limited part mounting space, it may be important to place the various parts in appropriate positions.

The wearable electronic device may include a plurality of electrodes for measuring biometric information related to electrocardiogram and/or body fat as a part of a biosensor module. At least some of the plurality of electrodes may be exposed outside the electronic device so as to be brought into contact with a user's wrist. The plurality of electrodes may be electrically connected with a substrate through a conductive member due to a limited mounting space inside the electronic device. In an example, the CN 110 794 666 A discloses a smart watch having a wireless charging module including a charging coil. The smart watch further includes an elastic sheet welded on an FPC, wherein a protrusion of the electrode is pressed against the elastic sheet during assembly to provide an electrical connection between the electrode and the FPC. In this case, a gap and a defect in contact may occur between the conductive member and the substrate due to an external force during assembly and handling of the electronic device.

### SUMMARY

Embodiments of the disclosure provide an electronic device including a plurality of electrical signal paths to strengthen an electrical connection structure of a plurality of electrodes.

However, the problems addressed by the disclosure are not limited to the aforementioned problems and may be expanded in various ways without departing from the spirit and scope of the disclosure.

An electronic device according to the invention is disclosed in the appended set of claims.

According to various example embodiments of the disclosure, the electrical connection structure between the plurality of electrodes, at least some of which are exposed outside the electronic device to measure biometric information, and the substrate disposed inside the electronic device may be strengthened.

In addition, the disclosure may provide various effects that are directly or indirectly recognized.

### BRIEF DESCRIPTION OF THE DRAWINGS

The above and other aspects, features and advantages of certain embodiments of the present disclosure will be more apparent from the following detailed description taken in conjunction with the accompanying drawings, in which:
FIG. 1 is a front perspective view of an electronic device according to various embodiments;
FIG. 2 is a rear perspective view of the electronic device according to various embodiments;
FIG. 3 is an exploded perspective view of the electronic device according to various embodiments;
FIG. 4 is a sectional view of the electronic device according to various embodiments;
FIG. 5 is an exploded perspective view illustrating a back plate, a circuit board, a wireless charging module, and an optical sensor module of the electronic device according to various embodiments;
FIG. 6 is an exploded side view of the back plate, the circuit board, the wireless charging module, and the optical sensor module of the electronic device according to various embodiments;
FIG. 7 is an exploded perspective view of a cover, the wireless charging module, and the optical sensor module of the electronic device according to various embodiments;
FIG. 8 is an exploded perspective view of the cover, the wireless charging module, and the optical sensor module of the electronic device according to various embodiments;
FIG. 9 is a diagram illustrating the wireless charging module of the electronic device according to various embodiments;
FIG. 10 is a perspective view illustrating a state in which the wireless charging module and the optical sensor module of the electronic device are coupled according to various embodiments;
FIG. 11 is a diagram illustrating the wireless charging module and the optical sensor module of the electronic device according to various embodiments;
FIG. 12 is a sectional view of the cover, the wireless charging module, and the optical sensor module of the electronic device according to various embodiments;
FIG. 13 is a perspective view illustrating the wireless charging module of the electronic device according to various embodiments; and
FIG. 14 is a block diagram illustrating an example electronic device in a network environment according to various embodiments.

With regard to the description of the drawings, identical or similar reference numerals may be used to refer to identical or similar components.

### DETAILED DESCRIPTION

Hereinafter, various example embodiments of the disclosure may be described with reference to accompanying drawings. Accordingly, those of ordinary skill in the art will recognize that various modifications, equivalents, and/or alternatives on the various example embodiments described herein can be variously made without departing from the scope and spirit of the disclosure.

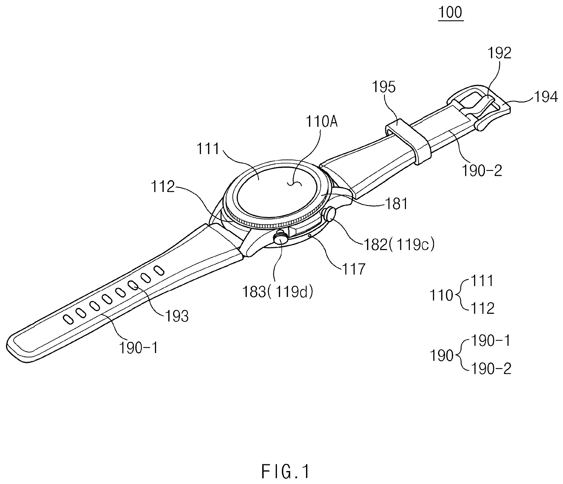

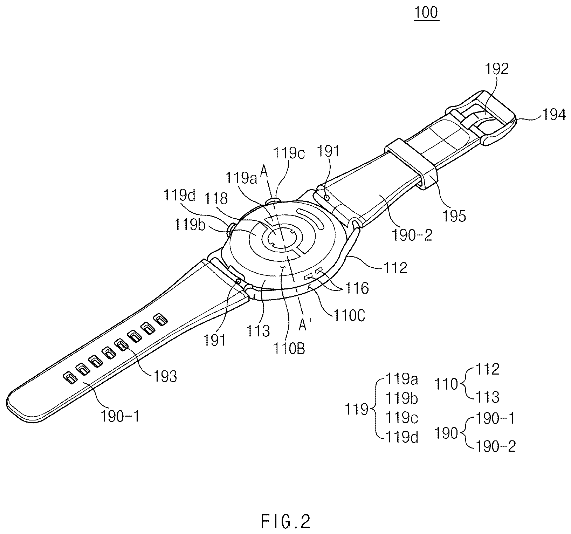

FIG. 1 is a front perspective view of an electronic device according to various embodiments. FIG. 2 is a rear perspective view of the electronic device according to various embodiments.

Referring to FIGS. 1 and 2, the electronic device 100 according to an embodiment may include a housing 110, audio modules (e.g., including audio circuitry) 116 and 117, sensor modules (e.g., including at least one sensor) 118 and 119, key input devices 181, 182, and 183, and a fastening member (e.g., a strap) 190.

The electronic device 100 according to an embodiment may be a wearable electronic device. For example, the electronic device 100 may be a watch type electronic device (e.g., a smart watch) wearable on a part of a user's body (e.g., a wrist or an ankle). However, the electronic device according to various embodiments of the disclosure is not limited to the illustrated embodiment.

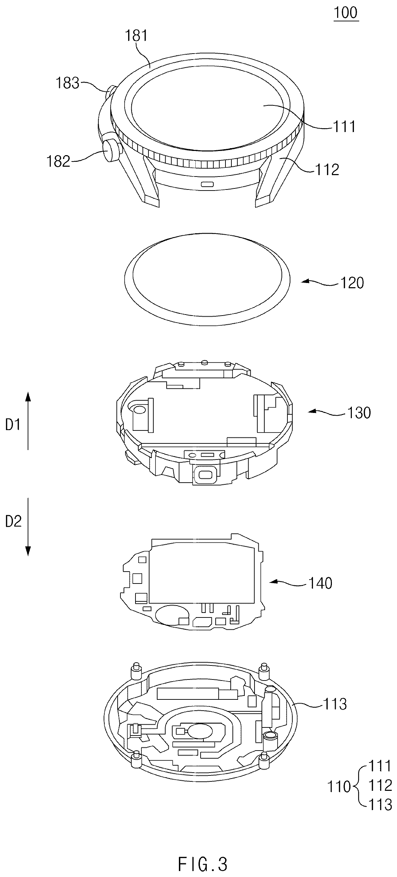

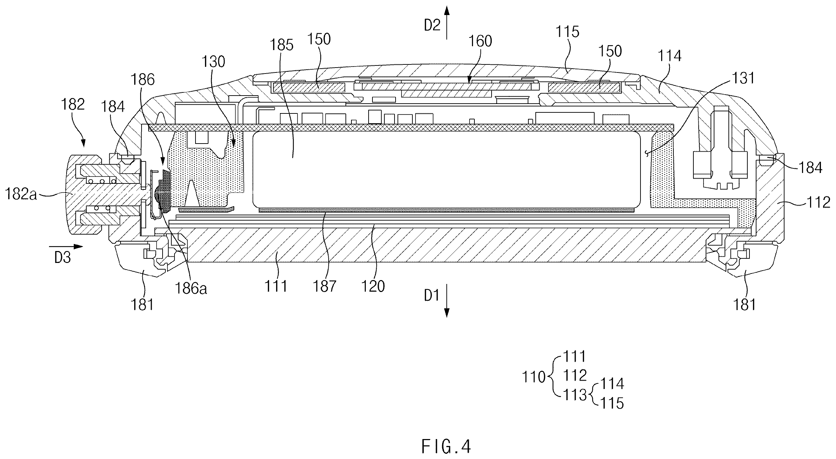

The housing 110 may form at least a portion of the exterior of the electronic device 100. The housing 110 may include a front plate 111, a side frame 112 (e.g., a side bezel or a side member), and a back plate 113. The front plate 111, the side frame 112, and the back plate 113 may be coupled together. For example, the housing 110 may form, through a coupling structure of the front plate 111, the side frame 112, and the back plate 113, an inner space in which other components of the electronic device 100 (e.g., a display 120, a bracket 130, a circuit board 140, and/or a battery 185 of FIGS. 3 and 4) are accommodated.

The housing 110 may include a front surface 110A, a rear surface 110B facing away from the front surface 110A, and a side surface 110C surrounding a space between the front surface 110A and the rear surface 110B. For example, the front surface 110A may be a surface formed by the front plate 111, the side surface 110C may be a surface formed by the side frame 112, and the rear surface 110B may be a surface formed by the back plate 113. According to various embodiments, the housing 110 may be understood to refer to a structure that forms some of the front surface 110A, the rear surface 110B, and the side surface 110C.

The front plate 111 may form at least a portion of the front surface 110A of the housing 110. For example, at least a portion of the front surface 110A may be formed by the substantially transparent front plate 111. The front plate 111 may include a glass plate including various coating layers, or a polymer plate.

The back plate 113 may form at least a portion of the rear surface 110B of the housing 110. For example, the rear surface 110B may be formed by the substantially opaque back plate 113. The back plate 113 may be formed of coated or colored glass, ceramic, a polymer, metal (e.g., aluminum, stainless steel (STS), or magnesium), or a combination of at least two of the aforementioned materials. In various embodiments, at least a partial region of the back plate 113 may be formed to be substantially transparent such that a portion of the sensor module 118 is visually exposed.

The side frame 112 may form at least a portion of the side surface 110C of the housing 110. For example, the side surface 110C may be formed by the side frame 112 coupled with the front plate 111 and the back plate 113. The side frame 112 may include metal and/or a polymer. In various embodiments, the side frame 112 may be integrally formed with the back plate 113. For example, the back plate 113 and the side frame 112 may be integrally formed with each other and may include the same material (e.g., a metallic material such as aluminum).

The electronic device 100 may include a display (e.g., the display 120 of FIGS. 3 to 4) disposed in the housing 110 and visually exposed (e.g., visible - as used herein, the terms "exposed" and "visible" may be used interchangeably and include being visible through a cover or protective layer or plate) outside the electronic device 100. For example, at least a portion of the display 120 may be visually exposed on the front surface 110A of the housing 110 through the front plate 111 formed to be substantially transparent. The display 120 may be formed in a shape corresponding to the shape of the front plate 111. For example, the display 120 may have various shapes such as a circular shape, an oval shape, or a polygonal shape. The display 120 may be combined with, or disposed adjacent to, touch detection circuitry, a pressure sensor capable of measuring the intensity (pressure) of a touch, and/or a fingerprint sensor.

The audio modules 116 and 117 may include the microphone hole 117 and the speaker hole 116. For example, a microphone for obtaining external sound may be disposed in the microphone hole 117. In various embodiments, the electronic device 100 may include a plurality of microphones to detect sounds in various directions. For example, a speaker for outputting sound to the outside may be disposed in the speaker hole 116 and may be used as an external speaker and a receiver for telephone call. In various embodiments, the speaker hole 116 and the microphone hole 117 may be implemented as a single hole. Alternatively, the electronic device 100 may be configured such that a speaker is included without the speaker hole 116 (e.g., a piezoelectric speaker).

The sensor modules 117, 118, and 119 may generate an electrical signal or a data value that corresponds to an operational state inside the electronic device 100 or an environmental state external to the electronic device 100. For example, the sensor modules 117, 118, and 119 may include the first biosensor module 118 and the second biosensor module 119.

The first biosensor module 118 may be disposed on the rear surface 110B of the housing 110. The first biosensor module 118 may be a sensor for obtaining biometric information of the user related to a heart rate and/or blood oxygen saturation. For example, the first biosensor module 118 may include a photoplethysmogram sensor (that is, a PPG sensor). The first biosensor module 118 may be disposed in the housing 110, and at least a portion of the first biosensor module 118 may be visually exposed on the rear surface 110B of the housing 110 through a partial region of the back plate 113.

The second biosensor module 119 may be disposed on the rear surface 110B of the housing 110. The second biosensor module 119 may be a sensor for obtaining biometric information of the user related to an electrocardiogram and a body composition (e.g., a body fat mass). For example, the second biosensor module 119 may include an electrical heart rate sensor (that is, an electrocardiogram (ECG) sensor) and/or a bioelectrical impedance analysis sensor (e.g., a bioelectrical impedance analysis (BIA) sensor). The second biosensor module 119a and 119b may include a first electrode region 119a, a second electrode region 119b, a third electrode region 119c, and a fourth electrode region 119d. The first electrode region 119a and the second electrode region 119b may include conductive regions formed on a surface of the back plate 113. The third electrode region 119c and the fourth electrode region 119d may be portions of the button members 182 and 183 formed of a conductive material. The first electrode region 119a and the second electrode region 119b may be brought into contact with the user's body (e.g., a wrist) when the user has the device on. The second biosensor module 119 may be configured to obtain an electrical signal from a part of the user's body through the first electrode region 119a to the fourth electrode region 119d brought into contact with the user's body and detect biometrical information of the user based on the obtained electrical signal.

In various embodiments, the electronic device 100 may further include another sensor module, for example, at least one of a gesture sensor, a gyro sensor, an atmospheric pressure sensor, a magnetic sensor, an acceleration sensor, a grip sensor, a color sensor, an infrared (IR) sensor, a biosensor, a temperature sensor, a humidity sensor, or an illuminance sensor.

In an embodiment, the key input devices 181, 182, and 183 may include the wheel member 181 (e.g., a wheel key or a rotary bezel) disposed on the front surface 110A of the housing 110 and rotatable in at least one direction and/or the button members 182 and 183 (e.g., side keys) disposed on the side surface 110C of the housing 110.

The wheel member 181 may have a form (e.g., a circular frame) corresponding to the form of the front plate 111. For example, the wheel member 181 may be rotated by a user operation to receive user inputs for implementing various functions of the electronic device 100. In various embodiments, the wheel member 181 may be implemented in the form of a soft key on the display 120 rather than a physical key, or may not be included in the electronic device 100.

The button members 182 and 183 may be rotated and/or pressed by a user operation to receive user inputs for implementing various functions of the electronic device 100. The button members 182 and 183 may include the first button member 182 and the second button member 183. At least one of the first button member 182 or the second button member 183 may be an electrode button (e.g., the third electrode region 119c and the fourth electrode region 119d) capable of detecting biometric information (e.g., an electrocardiogram and/or a body composition) of the user depending on contact with a part of the user's body (e.g., a finger).

The fastening member (e.g., a strap) 190 may allow the electronic device 100 to be detachably worn on a part of the user's body (e.g., a wrist or an ankle). For example, the fastening member 190 may be connected to at least a portion of the housing 110 and may be detachably fastened in a state of surrounding a part of the user's body. For example, the fastening member 190 may include a first fastening member 190-1 and a second fastening member 190-2 that are coupled to opposite sides of the housing 110, respectively. The first fastening member 190-1 and the second fastening member 190-2 may be connected with or separated from each other.

The fastening member 190 may be formed in a band or strap form to surround a part of the user's body. For example, the fastening member 190 may be formed of woven fabric, leather, rubber, urethane, metal, ceramic, or a combination of at least two of the aforementioned materials and may be implemented in an integrated form or with a plurality of unit links that are movable relative to each other.

The fastening member 190 may be detachably connected to the housing 110. For example, the fastening member 190 may be detachably connected to at least a partial region of the housing 110 using a locking member 191. According to various embodiments of the disclosure, the electronic device 100 may include various types of fastening members 190, and the fastening members 190 may be replaced depending on the user's taste and/or preference.

The fastening member 190 may include a fixing member 192, fixing-member fastening holes 193, a band guide member 194, and/or a band fixing ring 195. For example, the fixing member 192 may be configured to fix the housing 110 and the fastening member 190 to a part of the user's body (e.g., a wrist or an ankle). The fixing-member fastening holes 193 may fix the housing 110 and the fastening member 190 to the part of the user's body to correspond to the fixing member 192. The band guide member 194 may be configured to limit a movement range of the fixing member 192 when the fixing member 192 is fastened to one of the fixing-member fastening holes 193. Accordingly, the fastening member 190 may be closely fastened around the part of the user's body. The band fixing ring 195 may limit a movement range of the fastening member 190 in the state in which the fixing member 192 is fastened to one of the fixing-member fastening holes 193.

In various embodiments, the electronic device 100 may not include at least one component (e.g., the key input devices 181, 182, and 183 or the sensor modules 118, 119a, and 119b) among the components illustrated in FIGS. 1 and 2, or may additionally include other component(s). For example, the electronic device 100 may further include a connector hole (not illustrated). The connector hole (not illustrated) may accommodate a connector (e.g., a USB connector) for transmitting and/or receiving power and/or data with an external electronic device, or may accommodate a connector (e.g., an earphone connector) for transmitting and/or receiving an audio signal with an external electronic device.

FIG. 3 is an exploded perspective view of the electronic device according to various embodiments. FIG. 4 is a sectional view of the electronic device according to various embodiments.

FIG. 4 is a sectional view of the electronic device taken along line A-A' illustrated in FIG. 2.

Referring to FIGS. 3 and 4, the electronic device 100 according to an embodiment may include the housing 110, the display 120, the bracket 130, the circuit board 140, a wireless charging module (e.g., including wireless charging circuitry) 150, an optical sensor module (e.g., including at least one optical sensor) 160 (e.g., the first biosensor module 118 of FIG. 2), the wheel member (e.g., including a wheel key) 181, the button members (e.g., including a button) 182 and 183, a sealing member (e.g., a seal) 184, the battery 185, and a contact member (e.g., including an electrical contact) 186.

FIGS. 3 and 4 are views in which the fastening member (e.g., the fastening member 190 of FIGS. 1 and 2) of the electronic device 100 is omitted. Some of the components of the electronic device 100 illustrated in FIGS. 3 and 4 may be identical or similar to the components of the electronic device 100 illustrated in FIGS. 1 and 2, and repetitive descriptions will hereinafter be omitted.

The housing 110 may include the front plate 111, the side frame 112, and the back plate 113. For example, the front plate 111, the side frame 112, and the back plate 113 may form an inner space in which the display 120, the bracket 130, the circuit board 140, the wireless charging module 150, the optical sensor module 160, and the battery 185 are accommodated.

The front plate 111 may face in a first direction D1, and the back plate 113 may face in a second direction D2 opposite to the first direction D1. The side frame 112 may be disposed to surround the space between the front plate 111 and the back plate 113. For example, the first direction D1 may be a front direction in which the display 120 is viewed from the outside of the electronic device 100, and the second direction D2 may be a rear direction. The side frame 112 may include an opening region (not illustrated), and the front plate 111 may be exposed through the opening region in the first direction D1. For example, the front plate 111 may be coupled with the side frame 112 such that at least a portion of the front plate 111 is located in the opening region. The back plate 113 may be coupled to the side frame 112 to face the front plate 111.

The back plate 113 may include a rear case 114 coupled to the side frame 112 and a cover 115 coupled to the rear case 114. For example, the back plate 113 may be formed by a coupling of the rear case 114 and the cover 115. A surface of the rear case 114 and a surface of the cover 115 may form the rear surface of the electronic device 100. For example, the cover 115 may form the rear surface of the electronic device 100 together with the rear case 114 and may be attached to at least a portion of the rear case 114.

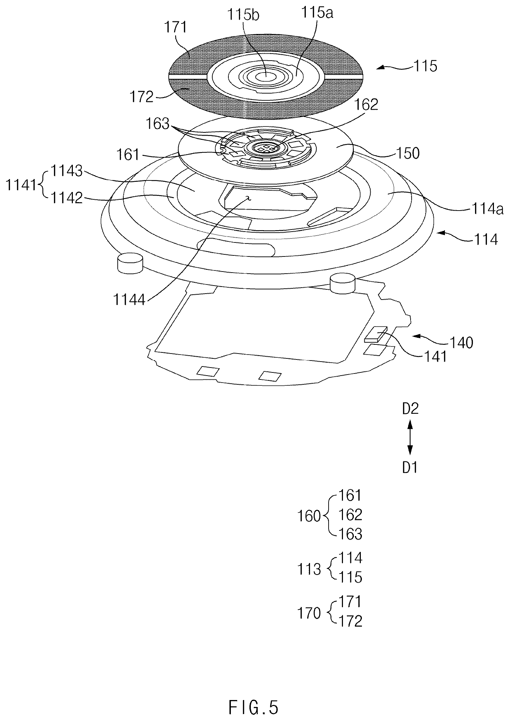

The back plate 113 may support the wireless charging module 150 and the optical sensor module 160. For example, the back plate 113 may be configured such that the wireless charging module 150 and the optical sensor module 160 are located in a space between the rear case 114 and the cover 115 (e.g., refer to FIG. 5). The wireless charging module 150 and the optical sensor module 160 may be attached to the cover 115. For example, the wireless charging module 150, the optical sensor module 160, and the cover 115 may be coupled to the rear case 114 in a state of being integrally attached and/or assembled.

The display 120 may be disposed between the front plate 111 and the bracket 130. The display 120 may be visually exposed (e.g., visible) through the front plate 111 in the front direction of the electronic device 100 (e.g., the first direction D1). For example, the display 120 may be attached to a rear surface (e.g., an inner surface or a surface facing in the second direction D2) of the front plate 111. The display 120 may be electrically connected to the circuit board 140. For example, the display 120 may be disposed to face the circuit board 140 with the bracket 130 therebetween, and a connector (not illustrated) of the display 120 may be connected to the circuit board 140 through an opening region (not illustrated) that is formed in the bracket 130.

The bracket 130 may be disposed in the housing 110 and may support other components of the electronic device 100 (e.g., a support plate 187, the circuit board 140, the contact member 186, and/or the battery 185). The bracket 130 may be assembled to the side frame 112 in the first direction D1. The bracket 130 may be surrounded by the side frame 112. For example, the bracket 130 may be connected to the side frame 112, or may be integrally formed with the side frame 112. The bracket 130 may be formed of a metallic material and/or a non-metallic (e.g., polymer) material.

The bracket 130 may be disposed between the circuit board 140 and the display 120. The bracket 130 may provide a battery receiving space 131 in which the battery 185 is accommodated. For example, the circuit board 140 may be disposed on one surface (e.g., a surface facing in the second direction D2) of the bracket 130, and the support plate 187 may be disposed on an opposite surface (e.g., a surface facing in the first direction D1) of the bracket 130. The support plate 187 may be disposed to face the display 120, and the battery 185 may be located between the circuit board 140 and the support plate 187 and may be stably fixed to the bracket 130 accordingly.

The circuit board 140 may be seated on the bracket 130. For example, the circuit board 140 may be disposed between the back plate 113 and the bracket 130. The circuit board 140 may be disposed to face the back plate 113 and may be disposed to face the display 120 with the bracket 130 therebetween. For example, the circuit board 140 may be located on one surface (e.g., a surface facing in the second direction D2) of the bracket 130.

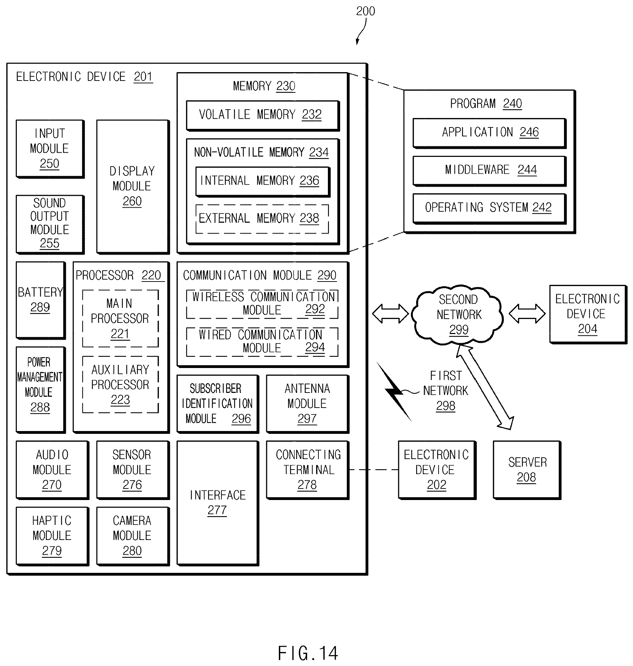

The circuit board 140 may have an electronic part located thereon, such as a processor (e.g., a processor 220 of FIG. 14), a memory (e.g., a memory 230 of FIG. 14), a communication module (e.g., a communication module 290 of FIG. 14), various types of sensor modules (e.g., a sensor module 276 of FIG. 14), an interface (e.g., an interface 277 of FIG. 14), or a connecting terminal (e.g., a connecting terminal 278 of FIG. 14). The processor may include, for example, one or more of a central processing unit, an application processor, a graphic processing unit (GPU), a sensor processor, or a communication processor, each of which may include various circuitry. The memory may include, for example, a volatile memory or a non-volatile memory. The interface may include, for example, a high definition multimedia interface (HDMI), a universal serial bus (USB) interface, an SD card interface, or an audio interface. The interface may electrically or physically connect the electronic device 100 with an external electronic device and may include a USB connector, an SD connector, an MMC connector, or an audio connector.

The wheel member (e.g., wheel key) 181 may be disposed on the front surface of the housing 110. The wheel member 181 may be disposed on the side frame 112 to surround the periphery of the front plate 111. The wheel member 181 may be coupled to the side frame 112 so as to be rotatable. For example, the wheel member 181 may be formed in a circular shape to correspond to the shape of the front plate 111 and/or the side frame 112. However, the shape of the wheel member 181 is not limited to the illustrated embodiment. In various embodiments, the electronic device 100 may sense and/or detect a rotation operation of the wheel member 181 and may execute various functions. For example, the electronic device 100 may be configured to change a screen displayed on the display 120 or adjust volume in a multimedia playback mode in response to rotation of the wheel member 181.

The button members (e.g., buttons) 182 and 183 may be disposed on the side surface of the housing 110. For example, the button members 182 and 183 may be used as input units for user inputs. The button members 182 and 183 may include the first button member 182 and the second button member 183. However, the number of button members 182 and 183 is not limited to the illustrated embodiment, and according to various embodiments, the electronic device 100 may not include one of the button members 182 and 183, or may additionally include another button member (e.g., a third button member (not illustrated)).

At least one of the button members 182 and 182 may be implemented with an electrode (e.g., the third electrode region 119c and the fourth electrode region 119d of FIGS. 1 and 2) for detecting a biometric signal of the user. For example, the first button member 182 and the second button member 183 may be used as electrode buttons for obtaining biometric information (e.g., an electrocardiogram and/or a body composition) of the user. As illustrated in FIG. 4, the first button member 182 may include an electrode part 182a formed of a conductive material. The electrode part 182a may be configured to be electrically connected with the circuit board 140 through the contact member 186. Although not illustrated in FIG. 4, the second button member 183 may have substantially the same structure as the first button member 182.

At least a portion of the electrode part 182a (e.g., the third electrode region 119c and the fourth electrode region 119d of FIGS. 1 and 2) may be exposed on the side surface of the housing 110 so as to be in contact with a part of the user's body (e.g., a finger). At least a portion of the electrode part 182a may be formed of a conductive material. The electrode part 182a may be configured to receive an electrical signal from the user's body and transmit the received electrical signal to control circuitry (e.g., the processor 220 of FIG. 14) disposed on the circuit board 140.

The button members 182 and 183 may function as a part of a sensor module for measuring an electrocardiogram and a body composition. For example, when the first button member 182 (e.g., the third electrode region 119c of FIGS. 1 and 2) and the second button member 183 (e.g., the fourth electrode region 119d of FIGS. 1 and 2) make contact with a finger of the user and the first electrode region (e.g., the first electrode region 119a of FIG. 2) and the second electrode region (e.g., the second electrode region 119b of FIG. 2) make contact with a wrist of the user, an electrical signal path may be formed between at least some of the electrode regions 119a, 119b, 119c, and 119d via the user's body, and the electronic device 100 may obtain biometric information related to an electrocardiogram and/or a body composition, based on the flow of the electrical signal.

The first button member 182 may be configured to press a switch 186a depending on a push operation by the user. The first button member 182 may operate the switch 186a by moving in a direction (e.g., a third direction D3) toward the switch 186a depending on the push operation of the user. For example, the first button member 182 may press the switch 186a of the contact member 186 depending on the push operation in the state of being brought into contact with a portion of the contact member 186. Although not illustrated in FIG. 4, the second button member 183 may also be configured to press a switch (not illustrated) depending on a user operation like the first button member 182.

The sealing member (e.g., a seal) 184 may be disposed between the back plate 113 and the side frame 112. For example, the sealing member 184 may seal a region where the rear case 114 of the back plate 113 and the side frame 112 make contact with each other in the state in which the back plate 113 and the side frame 112 are assembled. The sealing member 184 may block infiltration of foreign matter and/or moisture from outside the housing 110 through the space between the rear case 114 and the side frame 112.

The battery 185 may supply power to at least some of the components of the electronic device 100. The battery 185 may be supported by the bracket 130 and may be disposed in the housing 110 accordingly. For example, at least a portion of the battery 185 may be surrounded by the bracket 130.

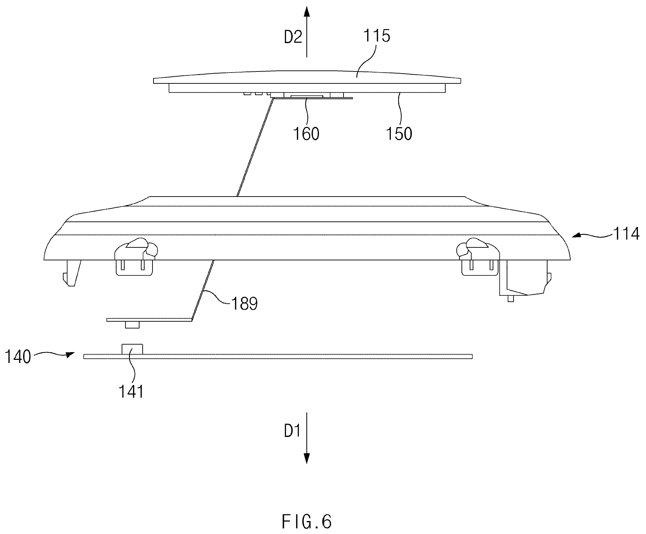

FIG. 5 is an exploded perspective view illustrating the back plate, the circuit board, the wireless charging module, and the optical sensor module of the electronic device according to various embodiments. FIG. 6 is an exploded view illustrating the back plate, the circuit board, the wireless charging module, and the optical sensor module of the electronic device according to various embodiments.

FIG. 5 is an exploded perspective view of the back plate, the circuit board, the wireless charging module, and the optical sensor module. FIG. 6 illustrates an assembly operation and an electrical connection structure of the back plate, the circuit board, the wireless charging module, and the optical sensor module.

Referring to FIGS. 5 and 6, the electronic device according to an embodiment (e.g., the electronic device 100 of FIGS. 1 to 4) may include the back plate 113, the circuit board 140, the wireless charging module (e.g., including wireless charging circuitry) 150, and the optical sensor module (e.g., including at least one optical sensor) 160. The components of the electronic device 100 illustrated in FIGS. 5 and 6 may be identical or similar to some of the components of the electronic device 100 illustrated in FIGS. 1 to 4, and repetitive descriptions may not be repeated.

The back plate 113 may include the rear case 114 and the cover 115. The cover 115 may be attached to at least a partial region of the rear case 114. The wireless charging module 150 and the optical sensor module 160 may be disposed between the rear case 114 and the cover 115.

The rear case 114 may include a first surface 114a that forms a portion of the rear surface of the electronic device 100 and a second surface (not illustrated) that faces away from the first surface 114a. The first surface 114a may be a surface facing in the second direction D2, and the second surface may be a surface facing in the first direction D1. For example, at least a portion of the first surface 114a may face the cover 115, and at least a portion of the second surface may face the circuit board 140.

The rear case 114 may include a seating portion 1141 on which the cover 115, the wireless charging module 150, and the optical sensor module 160 are disposed. The seating portion 1141 may be formed on at least a portion of the first surface 114a of the rear case 114. For example, at least a portion of the first surface 114a may be recessed to form the seating portion 1141. The seating portion 1141 may be formed in a shape corresponding to the cover 115.

The seating portion 1141 may include a first region 1142 connected with the first surface 114a in a stepped manner in the first direction D1 and a second region 1143 connected with the first region 1142 in a stepped manner in the first direction D1. For example, the first region 1142 may refer to a region surrounding the periphery of the second region 1143. The cover 115 may be disposed on the first region 1142, and the wireless charging module 150 and the optical sensor module 160 may be disposed on the second region 1143. For example, the cover 115 may be attached to at least a portion of the first region 1142, and the wireless charging module 150 may be attached to at least a portion of the second region 1143.

In an embodiment, an opening 1144 may be formed in a partial region of the seating portion 1141. A connecting member 189 that electrically and physically connects the wireless charging module 150 and the circuit board 140 may be located in the opening 1144. For example, as illustrated in FIG. 6, the connecting member (e.g., including a conductive material, e.g., including a wire) 189 may extend from the wireless charging module 150 to a first connector 141 of the circuit board 140 through the opening 1144 to connect the wireless charging module 150 and the circuit board 140 disposed with the rear case 114 therebetween.

The cover 115 may be attached with the wireless charging module 150 and the optical sensor module 160. The cover 115 may be coupled to the rear case 114 in a state in which the wireless charging module 150 and the optical sensor module 160 are attached to a surface of the cover 115 that faces in the first direction D1. For example, the cover 115, the wireless charging module 150, and the optical sensor module 160 may be coupled together and may be modularized as an integrated part accordingly.

At least a portion of the cover 115 may be formed to be transparent such that light passes for operation of the optical sensor module 160. The cover 115 may include transparent regions 115a and 115b formed in positions that correspond to a light emitting part 162 and a light receiving part 163 of the optical sensor module 160. For example, light generated from the light emitting part 162 of the optical sensor module 160 may pass through the first transparent region 115b and may reach an external object (e.g., a wrist of the user), and light reflected from the external object may pass through the second transparent region 115a and may reach the light receiving part 163.

A plurality of electrodes 170 (e.g., the second biosensor module 119 of FIG. 2) for measuring biometric information (e.g., an electrocardiogram and/or a body composition) of the user may be disposed on the cover 115. The plurality of electrodes 170 may be disposed to partially surround a surface of the cover 115. For example, the plurality of electrodes 170 may partially form the rear surface of the electronic device 100. The plurality of electrodes 170 may include a first electrode 171 and a second electrode 172, and the first electrode 171 and the second electrode 172 may be separated from each other. The first electrode 171 and the second electrode 172 may extend from a surface of the cover 115 that faces in the second direction D2 to a surface of the cover 115 that faces in the first direction D1. For example, each of the first electrode 171 and the second electrode 172 may include an outer electrode and an inner electrode. The outer electrodes of the first electrode 171 and the second electrode 172 may form a first electrode region (e.g., the first electrode region 119a of FIG. 2) and a second electrode region (e.g., the second electrode region 119b of FIG. 2) on the rear surface of the electronic device 100.

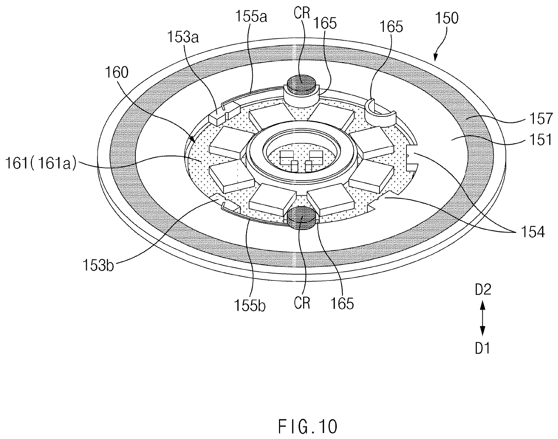

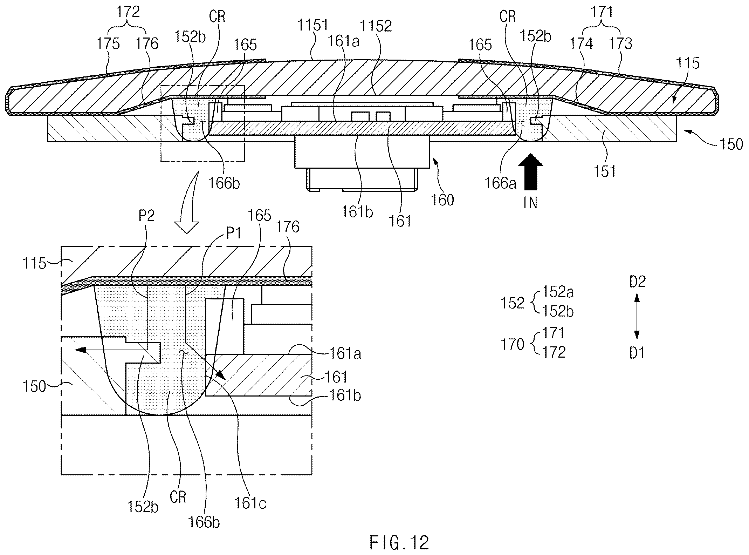

The plurality of electrodes 170 may be electrically connected to a sensor substrate 161 of the optical sensor module 160. For example, the plurality of electrodes 170 may be electrically connected to the circuit board 140 via the optical sensor module 160. The plurality of electrodes 170 and the sensor substrate 161 are electrically connected through a conductive member (e.g., a conductive resin or conductive silicone) that makes contact with the plurality of electrodes 170 and the sensor substrate 161. An electrical path between the plurality of electrodes 170 and the sensor substrate 161 includes a path (e.g., a first path P1 of FIG. 12) directly connected via the conductive member (e.g., a conductive resin CR of FIGS. 10, 11, and 12) and a path (e.g., a second path P2 of FIG. 12) connected from the conductive member via the wireless charging module 150. The electrical signal path between the plurality of electrodes 170 and the sensor substrate 161 will be described below in detail with reference to FIGS. 10, 11, and 12.

The wireless charging module 150 and the optical sensor module 160 may be coupled together to form a module assembly and may be disposed between the cover 115 and the rear case 114. The module assembly 150 and 160 may refer to a part into which the wireless charging module 150 and the optical sensor module 160 are integrated in a state of being electrically and physically connected with each other. For example, the module assembly 150 and 160 may be accommodated in a space between the seating portion 1141 of the rear case 114 and the cover 115. The module assembly 150 and 160 may be electrically connected to the circuit board 140 through the connecting member 189 that connects the wireless charging module 150 and the first connector 141. The connecting member 189 may include at least one of a flexible printed circuit board (FPCB), a wire or a cable.

The wireless charging module 150 may wirelessly receive power from an external electronic device (e.g., a wireless charging device). For example, the wireless charging module 150 may include a wireless charging coil (e.g., an Rx-coil) having a flat plate shape and may generate a current by electromagnetic induction generated from the external electronic device. The electronic device 100 may charge the battery (e.g., the battery 185 of FIG. 4) using the current generated from the wireless charging module 150. In various embodiments, the wireless charging module 150 may support one or more of various wireless charging methods including a magnetic resonance method or a magnetic induction method.

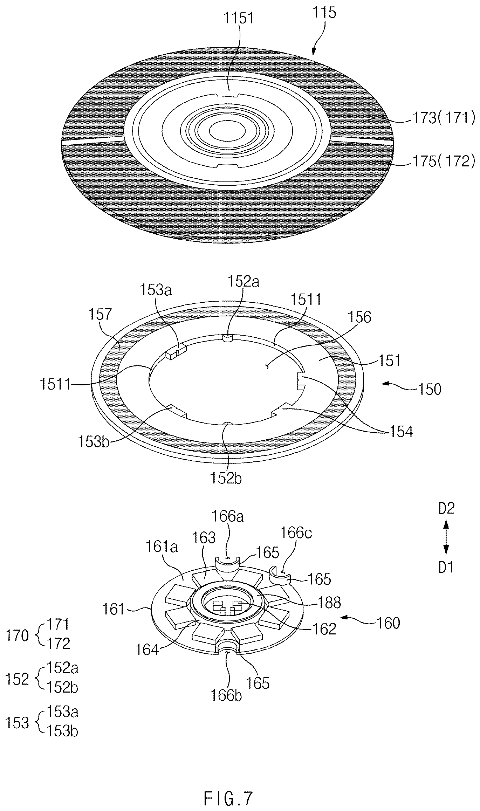

The wireless charging module 150 may be disposed to surround the optical sensor module 160. For example, the wireless charging module 150 may surround the sensor substrate 161 of the optical sensor module 160. The wireless charging module 150 may be formed in a disc shape having an open center such that the optical sensor module 160 is located therein. The wireless charging module 150 may be coupled to the sensor substrate 161 of the optical sensor module 160. For example, at least a portion of the wireless charging module 150 may be electrically and physically connected (e.g., soldered) with the sensor substrate 161 by making contact with the sensor substrate 161. A connection structure of the optical sensor module 160 and the wireless charging module 150 will be described below in detail with reference to FIGS. 7, 8, and 9.

The optical sensor module 160 (e.g., the first biosensor module 118 of FIG. 2) may measure a biometric signal of the user. For example, the optical sensor module 160 may measure the heart rate and blood oxygen saturation of the user. The optical sensor module 160 may be at least partially surrounded by the wireless charging module 150. For example, the optical sensor module 160 may be located inside the wireless charging module 150 to face a central region of the cover 115. The optical sensor module 160 may be disposed such that at least a portion thereof faces the transparent regions 115a and 115b of the cover 115.

The optical sensor module 160 may include the sensor substrate (e.g., sensor PCB) 161, the light emitting part 162, and the light receiving part 163. The light emitting part 162 and the light receiving part 163 may be disposed on one surface of the sensor substrate 161. For example, the light emitting part 162 and the light receiving part 163 may be disposed on one surface (e.g., a surface facing in the second direction D2) of the sensor substrate 161 that faces the cover 115. The light emitting part 162 and the light receiving part 163 may be disposed to be aligned with the transparent regions 115a and 115b of the cover 115. For example, the light emitting part 162 may face the first transparent region 115b, and the light receiving part 163 may face the second transparent region 115a.

The light emitting part 162 may emit light to the outside, and the light receiving part 163 may receive reflected light corresponding to the light emitted from the light emitting part 162. For example, the light emitting part 162 may be disposed on one surface (e.g., the surface facing in the second direction D2) of the sensor substrate 161 and may emit light in the second direction D2. The light emitting part 162 may emit light toward an external object (e.g., the user's body) making contact with the cover 115, and the light receiving part 163 may receive reflected light that returns by reflection of the emitted light by the external object.

The optical sensor module 160 may measure biometric information of the user by measuring the amount of transmitted light using an optical sensor. For example, light generated from the light emitting part 162 may reach the user's body through the cover 115, and the light receiving part 163 may receive reflected light that returns by reflection of at least a portion of the light emitted from the light emitting part 162 by a blood flow in a blood vessel of the user. For example, the optical sensor module 160 may be implemented with a photoplethysmogram (PPG) sensor.

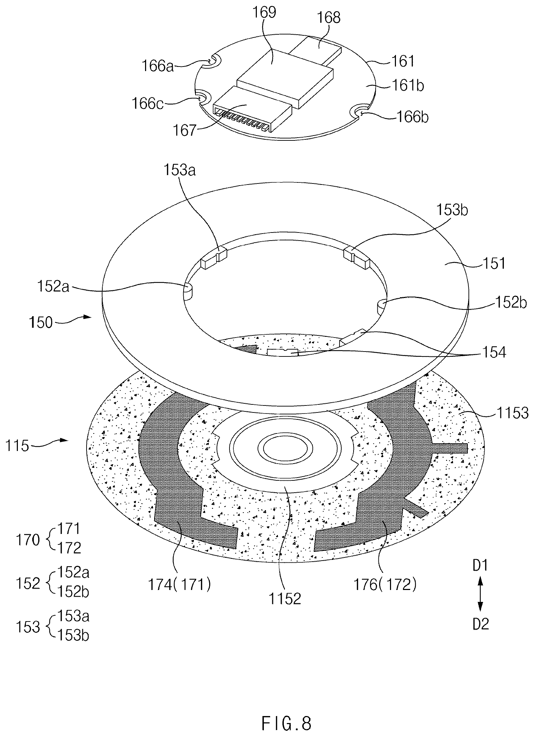

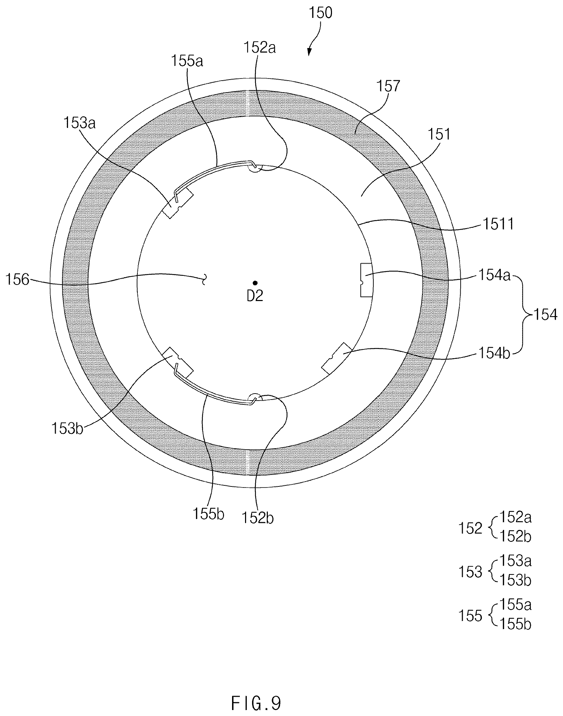

FIG. 7 is an exploded perspective view of the cover, the wireless charging module, and the optical sensor module of the electronic device according to various embodiments. FIG. 8 is an exploded perspective view of the cover, the wireless charging module, and the optical sensor module of the electronic device according to various embodiments. FIG. 9 is a diagram illustrating the wireless charging module of the electronic device according to various embodiments.

Referring to FIGS. 7, 8, and 9, the electronic device according to an embodiment (e.g., the electronic device 100 of FIGS. 1 to 4) may include the cover 115, the wireless charging module (e.g., including wireless charging circuitry) 150, and the optical sensor module (e.g., including at least one optical sensor) 160.

The cover 115 may include an outer surface 1151 facing in the second direction D2 and an inner surface 1152 facing away from the outer surface 1151. For example, the outer surface 1151 may form a portion of the rear surface (e.g., the rear surface 110B of FIG. 2) of the electronic device 100, and the inner surface 1152 may be at least partially attached to the rear case (e.g., the rear case 114 of FIGS. 5 and 6) and may not be exposed outside the electronic device 100. The inner surface 1152 of the cover 115 may face the wireless charging module 150 and the optical sensor module 160, and the outer surface 1151 of the cover 115 may at least partially make contact with the user's body (e.g., a wrist) when the electronic device 100 is worn.

The plurality of electrodes 170 may be disposed on the surfaces of the cover 115. The plurality of electrodes 170 may include a conductive material and may be formed to partially surround the surfaces 1151 and 1152 of the cover 115. For example, the plurality of electrodes 170 may be formed through a process of depositing the conductive material on the surfaces 1151 and 1152 of the cover 115. However, without being limited thereto, the plurality of electrodes 170 may be formed using various processes.

The plurality of electrodes 170 may include the first electrode 171 and the second electrode 172 separated from each other. For example, the first electrode 171 and the second electrode 172 may be spaced apart from each other so as to be electrically isolated from each other. The first electrode 171 and the second electrode 172 include outer electrodes 173 and 175 and inner electrodes 174 and 176 connected with the outer electrodes 173 and 175, respectively. The inner electrodes 174 and 176 and the outer electrodes 173 and 175 are electrically connected. The inner electrodes 174 and 176 may be formed on the inner surface 1152 of the cover 115, and the outer electrodes 173 and 175 may extend from the inner electrodes 174 and 176 to the outer surface 1151 of the cover 115 through a side surface of the cover 115 (e.g., a surface connecting the inner surface 1152 and the outer surface 1151). For example, it may be understood that the inner electrodes 174 and 176 refer to some of the electrodes formed on the inner surface 1152 of the cover 115 among the plurality of electrodes 170 and the outer electrodes 173 and 175 refer to the rest other than the inner electrodes 174 and 176.

The first electrode 171 may include the first outer electrode 173 and the first inner electrode174 extending from the first outer electrode 173, and the second electrode 172 may include the second outer electrode 175 and the second inner electrode 176 extending from the second outer electrode 175. The first outer electrode 173 and the second outer electrode 175 may be spaced apart from each other, and the first inner electrode 174 and the second inner electrode 176 may be spaced apart from each other. For example, the first outer electrode 173 may form a first electrode region (e.g., the first electrode region 119a of FIG. 2) on the rear surface of the electronic device 100 (e.g., the rear surface 110B of FIG. 2), and the second outer electrode 175 may form a second electrode region (e.g., the second electrode region 119b of FIG. 2) on the rear surface 110B of the electronic device 100.

The first electrode 171 and the second electrode 172 may be electrically connected to the sensor substrate 161 of the optical sensor module 160. For example, the first electrode 171 may be electrically connected with the sensor substrate 161 through contact between the first inner electrode 174 and a conductive member injected into a first recess 166a of the sensor substrate 161. The second electrode 172 may be electrically connected with the sensor substrate 161 through contact between the second inner electrode 176 and a conductive member injected into a second recess 166b of the sensor substrate 161. When the inner surface 1152 of the cover 115 is viewed from above, the first inner electrode 174 may partially overlap the first recess 166a, and the second inner electrode 176 may partially overlap the second recess 166b. The first electrode 171 and the second electrode 172 may be configured to transmit and/or receive an electrical signal with an electric element 168 mounted on a second surface 161b of the sensor substrate 161. For example, the electric element 168 may be a biosensor and may be an IC into which a PPG sensor, an ECG sensor, and/or a BIA sensor are integrated.

According to the embodiment illustrated in FIG. 8, a coating layer 1153 may be formed on the inner surface 1152 of the cover 115. For example, the coating layer 1153 may be formed to partially overlap the inner electrodes 174 and 176 in the state in which the inner electrodes 174 and 176 are disposed on the inner surface 1152 of the cover 115. The coating layer 1153 may be formed on the remaining portion other than at least a portion of the inner electrodes 174 and 174 and a portion corresponding to the optical sensor module 160. In FIG. 8, it may be understood that portions of the inner electrodes 174 and 176 connected with the outer electrodes 173 and 175 are hidden by the coating layer 1153.

The wireless charging module 150 may have, in a central portion thereof, an opening 156 having a shape corresponding to the sensor substrate 161. The wireless charging module 150 may include a base part 151 having the opening 156 formed in a central portion thereof and a plurality of pads 152, 153, and 154 extending from the base part 151. The opening 156 of the base part 151 may be aligned with the optical sensor module 160 in the first direction D1 and/or the second direction D2. An adhesive member 157 for attaching the cover 115 and the base part 151 may be disposed on one surface (e.g., a surface facing in the second direction D2) of the base part 151 that faces the cover 115.

The plurality of pads 152, 153, and 154 may extend from an inner periphery 1151 of the base part 151 that forms the opening 156. For example, the plurality of pads 152, 153, and 154 may extend or protrude from the inner periphery 1511 of the base part 151 toward the center of the opening 156. The plurality of pads 152, 153, and 154 may include the first pad 152, the second pad 153 electrically connected with the first pad 152, and the third pad 154 electrically isolated from the first pad 152 and the second pad 153. The first pad 152, the second pad 153, and the third pad 154 may be spaced apart from each other along the inner periphery 1151 of the base part 151. The first pad 152 and the second pad 153 may be connected through wiring 155 formed on the base part 151. For example, the base part 151 may be a printed circuit board (PCB), and the wiring 155 may be a pattern formed on the PCB.

The first pad 152 may be brought into contact with the conductive members that fill the first recess 166a and the second recess 166b (e.g., refer to FIGS. 10 to 12). The first pad 152 may include a first sub-pad 152a corresponding to the first recess 166a and a second sub-pad 152b corresponding to the second recess 166b. The first sub-pad 152a may be formed in a position facing the first recess 166a, and the second sub-pad 152b may be formed in a position facing the second recess 166b. As illustrated in FIG. 8, when the second surface 161b of the sensor substrate 161 is viewed from above, the first sub-pad 152a may at least partially overlap the first recess 166a and the first inner electrode 174, and the second sub-pad 152b may at least partially overlap the second recess 166b and the second inner electrode 176.

The second pad 153 is brought into contact with and coupled with the sensor substrate 161 (e.g., refer to FIGS. 10 to 12). For example, the second pad 153 may be physically and electrically connected (e.g., soldered) to the sensor substrate 161. The second pad 153 may include a third sub-pad 153a connected with the first sub-pad 152a through a first wire 155a and a fourth sub-pad 153b connected with the second sub-pad 152b through a second wire 155b. The first pad 152 is electrically connected with the sensor substrate 161 through the wiring 155 and the second pad 153. For example, an electrical signal path may be formed between the first sub-pad 152a and the sensor substrate 161 via the first wire 155a and the third sub-pad 153a. An electrical signal path may be formed between the second sub-pad 152b and the sensor substrate 161 via the second wire 155b and the fourth sub-pad 153b.

The third pad 154 may be brought into contact with and coupled with the sensor substrate 161 (e.g., refer to FIGS. 10 to 12). The third pad 154 may electrically connect the wireless charging module 150 to the sensor substrate 161 for a charging function of the wireless charging module 150. For example, the third pad 154 may be physically and electrically connected (e.g., soldered) to the sensor substrate 161. The third pad 154 may include a fifth sub-pad 154a and a sixth sub-pad 154b spaced apart from each other. The connection between the wireless charging module 150 and the sensor substrate 161 through the third pad 154 is irrelevant to connection for an operation/function of the optical sensor module 160 and an operation/function of the plurality of electrodes 170.

The optical sensor module 160 may include the sensor substrate 161, the light emitting part 162, the light receiving part 163, a first extension 164, and a second extension 165. For example, the light emitting part 162, the light receiving part 163, the first extension 164, and the second extension 165 may be disposed or formed on one surface of the sensor substrate 161 that faces the cover 115.

The sensor substrate 161 may include a first surface 161a facing the cover 115 and the second surface 161b facing away from the first surface 161a. For example, the first surface 161a may face in the second direction D2, and the second surface 161b may face in the first direction D1. The light emitting part 162, the light receiving part 163, and the extensions 164 and 165 may be disposed on the first surface 161a of the sensor substrate 161. For example, the light emitting part 162, the light receiving part 163, and the extensions 164 and 165 may be located between the first surface 161a and the cover 115. The electric element 168, a second connector 167, and a magnetic member (e.g., a magnet) 169 may be disposed on the second surface 161b of the sensor substrate 161. A connecting member (e.g., the connecting member 189 of FIG. 6) may be connected to the second connector 167 of the sensor substrate 161. For example, the connecting member 189 may be connected to the second connector 167 of the sensor substrate 161 and a first connector (e.g., the first connector 141 of FIG. 6) of a circuit board (e.g., the circuit board 140 of FIG. 6). The magnet 169 may be a magnet for attachment of a wireless charging device.

The sensor substrate 161 may be coupled (or, bonded) with the second pad 153 and the third pad 154 of the wireless charging module 150. For example, the second pad 153 and the third pad 154 may be disposed on the periphery of the first surface 161a of the sensor substrate 161 in an overlapping manner, and the sensor substrate 161 may be coupled with the second pad 153 and the third pad 154 through various coupling methods (e.g., laser soldering). The sensor substrate 161 may be electrically and physically connected with the wireless charging module 150 through the second pad 153 and the third pad 154.

A plurality of recesses 166a, 166b, and 166c may be formed at the periphery of the sensor substrate 161. The plurality of recesses 166a, 166b, and 166c may have a shape that is open at one side and may penetrate the first surface 161a and the second surface 161b of the sensor substrate 161. For example, at least portions of the periphery of the sensor substrate 161 may be concavely recessed to form the plurality of recesses 166a, 166b, and 166c. The plurality of recesses 166a, 166b, and 166c may include the first recess 166a formed in a position corresponding to the first inner electrode 174 of the first electrode 171 and the second recess 166b formed in a position corresponding to the second inner electrode 176 of the second electrode 172. For example, when the second surface 161b of the sensor substrate 161 is viewed from above, the first recess 166a may overlap at least a portion of the first inner electrode 174, and the second recess 166b may overlap at least a portion of the second inner electrode 176.

Although not illustrated in FIGS. 7 and 8, the conductive members (not illustrated) (e.g., the conductive resin CR of FIGS. 10, 11, and 12) for electrically connecting the sensor substrate 161 and the inner electrodes 174 and 176 may fill the first recess 166a and the second recess 166b. For example, the conductive members may be brought into contact with side surfaces (e.g., a side surface 161c of FIG. 12) within the first and second recesses 166a and 166b among the side surfaces of the sensor substrate 161 and the plurality of electrodes 170 (e.g., the inner electrodes 174 and 176) to form an electrical path between the plurality of electrodes 170 and the sensor substrate 161. The side surface 161c of the sensor substrate 161 may refer to a surface that connects the first surface 161a and the second surface 161b.

The plurality of recesses 166a, 166b, and 166c may further include the third recess 166c formed in a position spaced apart from the first recess 166a and the second recess 166b. An adhesive material for attaching the cover 115, the sensor substrate 161, and the wireless charging module 150 may fill the third recess 166c. For example, the adhesive material may include epoxy. However, without being limited thereto, the third recess 166c and the adhesive material may be omitted.

The light emitting part 162 may be disposed on the first surface 161a of the sensor substrate 161 and may emit light toward the cover 115. The light emitting part 162 may be disposed on a central region of the first surface 161a. The light emitting part 162 may include one or more light emitting elements. For example, the light emitting part 162 may include an LED. The light emitting part 162 may emit light having various colors. The light emitted from the light emitting part 162 may have a wavelength range of about 380 nm to about 800 nm. The light emitting part 162 may be located inside the first extension 164 and may be spaced apart from the light receiving part 163.

The light receiving part 163 may be disposed on a peripheral region of the first surface 161a. The light receiving part 163 may be located outside the first extension 164 and may be spaced apart from the light emitting part 162 by a predetermined distance. The light receiving part 163 may receive at least a portion of reflected light that returns by reflection of light emitted from the light emitting part 162 by an external object. For example, the light receiving part 163 may include a photo diode (PD). For example, a plurality of light receiving parts 163 may be formed. The plurality of light receiving parts 163 may be radially disposed along the outside of the light emitting part 162 or the first extension 164. The number and positions of light receiving parts 163 are not limited to the illustrated embodiment and may be diversely changed.

The light emitting part 162 and the light receiving part 163 may be configured to be located in spaces that are separated from each other by the first extension 164 disposed between the light emitting part 162 and the light receiving part 163. For example, the light emitting part 162 may be located in a space formed by the first surface 161a of the sensor substrate 161, the cover 115, and an inner wall of the first extension 164, and the light receiving part 163 may be located in a space formed outside the first extension 164 by an outer wall of the first extension 164, the first surface 161a, and the cover 115.

The light emitting part 162 and the light receiving part 163 may be electrically connected with the sensor substrate 161. For example, the light emitting part 162 and the light receiving part 163 may be disposed on the first surface 161a so as to be electrically connected with the sensor substrate 161. In various embodiments, the light emitting part 162 and the light receiving part 163 may be disposed on the sensor substrate 161 in a chip on board (COB) manner. For example, elements (e.g., LED chips and/or PD chips) of the light emitting part 162 and the light receiving part 163 may be directly attached to the first surface 161a of the sensor substrate 161 using an adhesive member (e.g., Ag epoxy) formed of a conductive material and may be wire-bonded to the sensor substrate 161 using wires.

The first extension 164 may be disposed on the first surface 161a of the sensor substrate 161 to surround the periphery of the light emitting part 162. For example, the light emitting part 162 may be located inside the first extension 164. The first extension 164 may be located between the light emitting part 162 and the light receiving part 163. For example, when the first surface 161a of the sensor substrate 161 is viewed from above, the first extension 164 may be formed between the light emitting part 162 and the light receiving part 163 to surround the light emitting part 162. The first extension 164 may separate or isolate the light emitting part 162 and the light receiving part 163 from each other. The first extension 164 may function as a partition wall between the light emitting part 162 and the light receiving part 163. For example, the first extension 164 may block a path along which light emitted from the light emitting part 162 directly moves to the light receiving part 163 without travelling outside the electronic device 100 through the cover 115, thereby preventing/blocking the light receiving part 163 from receiving coherent light emitted from the light emitting part 162 other than reflected light.

The first extension 164 may include a magnetic member (e.g., a magnet). For example, the first extension 164 may be partially formed of a magnet. In the case in which the first extension 164 is formed of a magnet, the first extension 164 may interact with a magnet included in an external electronic device (e.g., a wireless charging device) to stably mount the electronic device 100 on the charging device and may align an antenna included in the wireless charging device and the wireless charging module 150 to allow the electronic device 100 to be located in a position in which charging is able to be performed.

The first extension 164 may protrude from the first surface 161a of the sensor substrate 161 by a predetermined height (e.g., a length extending in the second direction D2), and the cover 115 and the sensor substrate 161 may be spaced apart from each other at a predetermined interval to correspond to the height of the first extension 164. For example, the first extension 164 may space the cover 115 apart from the sensor substrate 161, thereby preventing and/or reducing collision and/or interference of the cover 115 with the light emitting part 162 and/or the light receiving part 163. The first extension 164 may be attached to the cover 115. For example, the first extension 164 may be attached with the cover 115 through at least one adhesive member 188 disposed between the first extension 164 and the cover 115.

The second extension 165 may be disposed on an edge portion of the first surface 161a of the sensor substrate 161. For example, the second extension 165 may be disposed along the peripheries of the plurality of recesses 166a, 166b, and 166c of the sensor substrate 161. The second extension 165 may be formed in a shape corresponding to the plurality of recesses 166a, 166b, and 166c so as to be connected with side surfaces within the plurality of recesses 166a, 166b, and 166c. The second extension 165 may include a plurality of second extensions 165 to correspond to the plurality of recesses 166a, 166b, and 166c. The second extensions 165 may protrude from the first surface 161a of the sensor substrate 161 by a predetermined height (e.g., a length extending in the second direction D2).

The plurality of second extensions 165 may be brought into contact with the materials (the conductive members or the adhesive material) that fill the plurality of recesses 166a, 166b, and 166c. For example, the second extensions 165 may increase the areas in contact with the conductive members and/or the adhesive material, and thus electrical and/or physical connection by the conductive members and/or the adhesive material may be stably maintained. The second extensions 165 corresponding to the first recess 166a and the second recess 166b may be formed of a metallic material, and the conductive members filling the first recess 166a and the second recess 166b may make electrical contact with the side surface of the sensor substrate 161 and the second extensions 165.

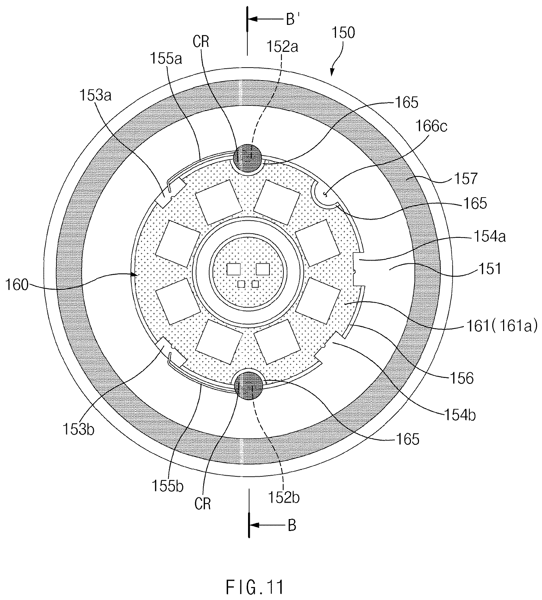

FIG. 10 is a perspective view illustrating a state in which the wireless charging module and the optical sensor module of the electronic device are coupled according to various embodiments. FIG. 11 is a diagram illustrating the wireless charging module and the optical sensor module of the electronic device according to various embodiments. FIG. 12 is a sectional view of the cover, the wireless charging module, and the optical sensor module of the electronic device according to various embodiments.

FIGS. 10 and 11 are views in which the cover is omitted. FIG. 12 illustrates a section of the wireless charging module and the optical sensor module taken along line B-B' illustrated in FIG. 11.

Referring to FIGS. 10, 11, and 12, in the electronic device according to an embodiment (e.g., the electronic device 100 of FIGS. 1 to 4), the cover 115, the wireless charging module 150, and the optical sensor module 160 may be integrally assembled by a coupling and/or attachment thereof. For example, FIGS. 10 and 11 may be views in which the cover 115 is omitted, and the cover 115 may be attached to the adhesive member 157 on the base part 151 of the wireless charging module 150 while facing the first surface 161a of the sensor substrate 161. In various embodiments, the cover 115, the wireless charging module 150, and the optical sensor module 160 may be coupled to the rear case 114 in the state of being assembled as illustrated in FIG. 12 (e.g., refer to FIG. 6).

The optical sensor module 160 may be disposed in the opening 156 of the wireless charging module 150 such that the first surface 161a of the sensor substrate 161 is brought into contact with the second pad 153 and the third pad 154. The first surface 161a of the sensor substrate 161 may face the cover 115. For example, the first surface 161a of the sensor substrate 161 may partially face the first inner electrode 174 and the second inner electrode 176 disposed on the cover 115.

The second pad 153 and the third pad 154 of the wireless charging module 150 may be coupled to the sensor substrate 161. The second pad 153 and the third pad 154 may be physically and electrically connected to the first surface 161a of the sensor substrate 161. For example, the second pad 153 and the third pad 154 may be bonded to the first surface 161a of the sensor substrate 161 through a laser soldering process. However, a method by which the second pad 153 and the third pad 154 are connected to the sensor substrate 161 is not limited to soldering. The optical sensor module 160 may be fixedly coupled to the inside of the opening 156 of the wireless charging module 150 as the second pad 153 and the third pad 154 are bonded to the sensor substrate 161.

The first pad 152 of the wireless charging module 150 may extend from the base part 11 toward the first recess 166a (e.g., the first recess 166a of FIGS. 7 and 8) and the second recess 166b (e.g., the second recess 166b of FIGS. 7 and 8) of the sensor substrate 161. The first pad 152 may include the first sub-pad 152a and the second sub-pad 152b. The first sub-pad 152a may face the first recess 166a, and the second sub-pad 152b may face the second recess 166b. The first sub-pad 152a and the second sub-pad 152b may be spaced apart from the first recess 166a and the second recess 166b. The conductive members may be disposed in the first recess 166a and the second recess 166b. For example, the conductive members may be formed of the conductive resin CR. The conductive resin CR may be disposed in the first recess 166a and the second recess 166b so as to be brought into contact with the first sub-pad 152a and the second sub-pad 152b. For example, the conductive resin CR may fill the first recess 166a and the second recess 166b while surrounding the first sub-pad 152a and the second sub-pad 152b.

The conductive resin CR may be brought into contact with the side surface 161c of the sensor substrate 161, the second extensions 165, the first pad 152, and the inner electrodes 174 and 176. In various embodiments, the conductive resin CR may include silicone (e.g., Ag silicone) having conductivity. For example, as illustrated in FIG. 12, the conductive resin CR may be formed by injecting (IN) liquid Ag silicone having a predetermined viscosity into the spaces between the first and second recesses 166a and 166b and the wireless charging module 150 in the state in which the cover 115, the optical sensor module 160, and the wireless charging module 150 are assembled and thereafter curing the liquid Ag silicone. The type of the conductive resin CR is not limited to the aforementioned example. Although not illustrated, an adhesive material (e.g., an epoxy adhesive) may be injected into the space between the third recess 166c and the wireless charging module 150.

The plurality of electrodes 170 are electrically connected with the sensor substrate 161 as the inner electrodes 174 and 176 are brought into contact with the conductive resin CR. For example, the first inner electrode 174 of the first electrode 171 may be brought into contact with the conductive resin CR filling the first recess 166a, and the second inner electrode 176 of the second electrode 172 may be brought into contact with the conductive resin CR filling the second recess 166b. A plurality of electrical signal paths may be formed between the plurality of electrodes 170 and the sensor substrate 161.

The plurality of electrical signal paths P1 and P2 may include the first electrical signal path P1 along which the inner electrodes 174 and 176 and the sensor substrate 161 are directly electrically connected through the conductive resin CR and the second electrical signal path P2 along which the inner electrodes 174 and 176 and the sensor substrate 161 are electrically connected from the conductive resin CR via the wireless charging module 150. For example, the first electrical signal path P1 may extend from the inner electrodes 174 and 176 to the sensor substrate 161 (e.g., the side surface 161c and/or the second extension 165) via the conductive resin CR. The second electrical signal path P2 may extend from the inner electrodes 174 and 176 to the sensor substrate 161 via the conductive resin CR, the first pad 152, the wiring 155, and the second pad 153.

The first electrode 171 and the second electrode 172 are connected with the sensor substrate 161 through the first electrical signal path P1 and the second electrical signal path P2, respectively, and the electrical signal path between the first electrode 171 and the sensor substrate 161 may be separated from the electrical signal path between the second electrode 172 and the sensor substrate 161. For example, the first electrical signal path between the first electrode 171 and the sensor substrate 161 may extend from the first outer electrode 173 to the sensor substrate 161 via the first inner electrode 174 and the conductive resin CR in the first recess 166a. The second electrical signal path between the first electrode 171 and the sensor substrate 161 may extend from the first inner electrode 174 to the sensor substrate 161 via the conductive resin CR in the first recess 166a, the first sub-pad 152a, the first wire 155a, and the third sub-pad 153a. For example, the first electrical signal path between the second electrode 172 and the sensor substrate 161 may extend from the second outer electrode 175 to the sensor substrate 161 via the second inner electrode 176 and the conductive resin CR in the second recess 166b. The second electrical signal path between the second electrode 172 and the sensor substrate 161 may extend from the second outer electrode 175 to the sensor substrate 161 via the second inner electrode 176, the conductive resin CR in the second recess 166b, the second sub-pad 152b, the second wire 155b, and the fourth sub-pad 153b.

Referring to FIG. 12, the plurality of electrodes 170 may be configured such that the inner electrodes 174 and 176 and the outer electrodes 173 and 175 are integrally formed with each other. For example, by depositing one conductive material to cover the outer surface 1151 and the inner surface 1152 of the cover 115, the plurality of electrodes 170 may be divided into a region forming the inner electrodes 174 and 176 and a region forming the outer electrodes 173 and 175. However, the shape and/or structure of the plurality of electrodes 170 are not limited to the illustrated embodiment. In various embodiments, the plurality of electrodes 170 may be configured such that the outer electrodes 173 and 175 and the inner electrodes 174 and 176 are separated from each other without being integrally formed with each other and may be electrically connected through a conductive material inserted into a through-hole (not illustrated) that is formed through the cover 115.

According to various embodiments of the disclosure, the two electrical signal paths P1 and P2 may be formed between the plurality of electrodes 170 and the sensor substrate 161. Accordingly, the electrical connection structure of the plurality of electrodes 170 may be strengthened, and a defect in connection may be improved. For example, in a case in which a defect in contact or a gap occurs between the conductive resin CR and the side surface of the sensor substrate 161 due to a movement between the wireless charging module 150 and the sensor substrate 161, the electrical connection of the plurality of electrodes 170 may be stably maintained through a path passing through the wireless charging module 150 (that is, the second electrical signal path P2).

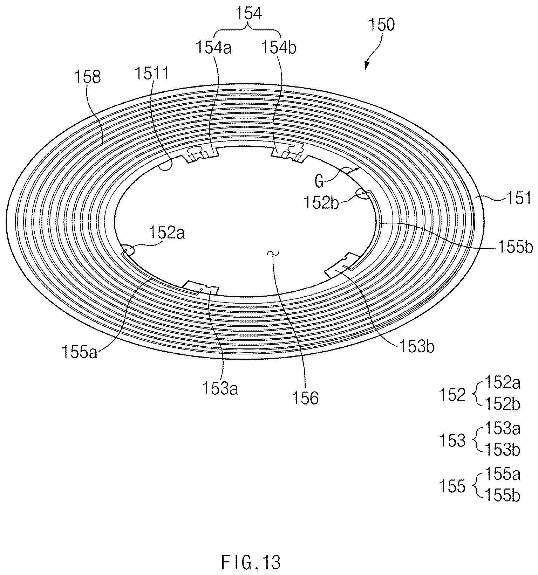

FIG. 13 is a perspective view illustrating the wireless charging module of the electronic device according to various embodiments.

Referring to FIG. 13, the wireless charging module 150 according to an embodiment may include the base part 151, the plurality of pads 152, 153, and 154, the wiring pattern 155 (e.g., the wiring 155 of FIGS. 9, 10, and 11), and a charging coil pattern 158. The plurality of pads 152, 153, and 154 may extend from the base part 151 and may include the first pad 152, the second pad 153, and the third pad 154. For example, the wireless charging module 150 may be a printed circuit board (PCB) or a flexible printed circuit board (FPCB) that has the opening 156 formed therein. The wiring pattern 155 and the charging coil pattern 158 may be conductive patterns formed on the PCB or the FPCB.

The charging coil pattern 158 may be formed on a partial region of the base part 151. The charging coil pattern 158 may be configured to interact with a coil (not illustrated) of an external electronic device located outside the electronic device 100 to supply power to the battery of the electronic device 100 (e.g., the battery 185 of FIGS. 3 and 4 and/or a battery 289 of FIG. 14) or a battery (not illustrated) of the external electronic device (e.g., an electronic device 202 or 204 of FIG. 14). The charging coil pattern 158 may be electrically connected with the third pad 154, and the third pad 154 may electrically connect the charging coil pattern 158 and the sensor substrate 161. For example, one end portion of the charging coil pattern 158 may be electrically connected with the fifth sub-pad 154a, and an opposite end portion of the charging coil pattern 158 may be electrically connected with the sixth sub-pad 154b. In various embodiments, the charging coil pattern 158 may be electrically connected with a charging circuit disposed on the circuit board 140 (e.g., a power management module 288 of FIG. 14) through electrical connection of the sensor substrate 161 and the circuit board (e.g., the circuit board 140 of FIGS. 3, 4, 5, and 6).

The charging coil pattern 158 may be formed in a spiral shape on a partial region of the base part 151. The charging coil pattern 158 may be formed to be spaced apart from the inner periphery 1511 of the base part 151 at a predetermined interval G. The interval G between the charging coil pattern 158 and the inner periphery 1511 may be a component for forming the wiring pattern 155. For example, the wiring pattern 155 connecting the first pad 152 and the second pad 153 may be formed between a portion of the charging coil pattern 158 closest to the inner periphery 1511 and the inner periphery 1511 so as not to overlap the charging coil pattern 158.

The wiring pattern 155 may electrically connect the first pad 152 and the second pad 153. The wiring pattern 155 may be formed on a region between the inner periphery 1511 of the base part 151 and the charging coil pattern 158 so as not to contact or overlap the charging coil pattern 158. For example, the wiring pattern 155 and the charging coil pattern 158 may be patterns for different functions and may be electrically isolated from each other.

The wiring pattern 155 may include the first conductive pattern 155a (e.g., the first wire 155a of FIGS. 9, 10, and 11) that electrically connects the first sub-pad 152a and the third sub-pad 153a and the second conductive pattern 155b (e.g., the second wire 155b of FIGS. 9, 10, and 11) that electrically connects the second sub-pad 152b and the fourth sub-pad 153b. Referring to FIGS. 10, 11, and 13 together, the third sub-pad 153a may be soldered to the sensor substrate (e.g., the sensor substrate 161 of FIGS. 10 and 11) and may be electrically connected with the sensor substrate 161, and the first sub-pad 152a may be electrically connected with the sensor substrate 161 through the first conductive pattern 155a and the third sub-pad 153a. The fourth sub-pad 153b may be soldered to the sensor substrate 161 and may be electrically connected with the sensor substrate 161, and the second sub-pad 152b may be electrically connected with the sensor substrate 161 through the second conductive pattern 155b and the fourth sub-pad 153b. However, a method of electrically/physically connecting the third sub-pad 153a and/or the fourth sub-pad 153b with the sensor substrate 161 is not limited to the soldering.

FIG. 14 is a block diagram illustrating an example electronic device in a network environment according to various embodiments.

Referring to FIG. 14, the electronic device 201 (e.g., the electronic device 100 of FIGS. 1 to 4) in the network environment 200 may communicate with an electronic device 202 via a first network 298 (e.g., a short-range wireless communication network), or at least one of an electronic device 204 or a server 208 via a second network 299 (e.g., a long-range wireless communication network). According to an embodiment, the electronic device 201 may communicate with the electronic device 204 via the server 208. According to an embodiment, the electronic device 201 may include a processor 220, memory 230, an input module 250, a sound output module 255, a display module 260, an audio module 270, a sensor module 276, an interface 277, a connecting terminal 278, a haptic module 279, a camera module 280, a power management module 288, a battery 289, a communication module 290, a subscriber identification module (SIM) 296, or an antenna module 297. In various embodiments, at least one of the components (e.g., the connecting terminal 278) may be omitted from the electronic device 201, or one or more other components may be added in the electronic device 201. In various embodiments, some of the components (e.g., the sensor module 276, the camera module 280, or the antenna module 297) may be implemented as a single component (e.g., the display module 260).

The processor 220 may execute, for example, software (e.g., a program 240) to control at least one other component (e.g., a hardware or software component) of the electronic device 201 coupled with the processor 220, and may perform various data processing or computation. According to an embodiment, as at least part of the data processing or computation, the processor 220 may store a command or data received from another component (e.g., the sensor module 276 or the communication module 290) in volatile memory 232, process the command or the data stored in the volatile memory 232, and store resulting data in non-volatile memory 234. According to an embodiment, the processor 220 may include a main processor 221 (e.g., a central processing unit (CPU) or an application processor (AP)), or an auxiliary processor 223 (e.g., a graphics processing unit (GPU), a neural processing unit (NPU), an image signal processor (ISP), a sensor hub processor, or a communication processor (CP)) that is operable independently from, or in conjunction with, the main processor 221. For example, when the electronic device 201 includes the main processor 221 and the auxiliary processor 223, the auxiliary processor 223 may be adapted to consume less power than the main processor 221, or to be specific to a specified function. The auxiliary processor 223 may be implemented as separate from, or as part of the main processor 221.

The auxiliary processor 223 may control at least some of functions or states related to at least one component (e.g., the display module 260, the sensor module 276, or the communication module 290) among the components of the electronic device 201, instead of the main processor 221 while the main processor 221 is in an inactive (e.g., sleep) state, or together with the main processor 221 while the main processor 221 is in an active state (e.g., executing an application). According to an embodiment, the auxiliary processor 223 (e.g., an image signal processor or a communication processor) may be implemented as part of another component (e.g., the camera module 280 or the communication module 290) functionally related to the auxiliary processor 223. According to an embodiment, the auxiliary processor 223 (e.g., the neural processing unit) may include a hardware structure specified for artificial intelligence model processing. An artificial intelligence model may be generated by machine learning. Such learning may be performed, e.g., by the electronic device 201 where the artificial intelligence is performed or via a separate server (e.g., the server 208). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 230 may store various data used by at least one component (e.g., the processor 220 or the sensor module 276) of the electronic device 201. The various data may include, for example, software (e.g., the program 240) and input data or output data for a command related thereto. The memory 230 may include the volatile memory 232 or the non-volatile memory 234.

The program 240 may be stored in the memory 230 as software, and may include, for example, an operating system (OS) 242, middleware 244, or an application 246.

The input module 250 may receive a command or data to be used by another component (e.g., the processor 220) of the electronic device 201, from the outside (e.g., a user) of the electronic device 201. The input module 250 may include, for example, a microphone, a mouse, a keyboard, a key (e.g., a button), or a digital pen (e.g., a stylus pen).

The sound output module 255 may output sound signals to the outside of the electronic device 201. The sound output module 255 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 260 may visually provide information to the outside (e.g., a user) of the electronic device 201. The display module 260 may include, for example, a display, a hologram device, or a projector and control circuitry to control a corresponding one of the display, hologram device, and projector. According to an embodiment, the display module 260 may include a touch sensor adapted to detect a touch, or a pressure sensor adapted to measure the intensity of force incurred by the touch.

The audio module 270 may convert a sound into an electrical signal and vice versa. According to an embodiment, the audio module 270 may obtain the sound via the input module 250, or output the sound via the sound output module 255 or a headphone of an external electronic device (e.g., an electronic device 202) directly (e.g., wiredly) or wirelessly coupled with the electronic device 201.

The sensor module 276 may detect an operational state (e.g., power or temperature) of the electronic device 201 or an environmental state (e.g., a state of a user) external to the electronic device 201, and then generate an electrical signal or data value corresponding to the detected state. According to an embodiment, the sensor module 276 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 277 may support one or more specified protocols to be used for the electronic device 201 to be coupled with the external electronic device (e.g., the electronic device 202) directly (e.g., wiredly) or wirelessly. According to an embodiment, the interface 277 may include, for example, a high definition multimedia interface (HDMI), a universal serial bus (USB) interface, a secure digital (SD) card interface, or an audio interface.

A connecting terminal 278 may include a connector via which the electronic device 201 may be physically connected with the external electronic device (e.g., the electronic device 202). According to an embodiment, the connecting terminal 278 may include, for example, a HDMI connector, a USB connector, a SD card connector, or an audio connector (e.g., a headphone connector).

The haptic module 279 may convert an electrical signal into a mechanical stimulus (e.g., a vibration or a movement) or electrical stimulus which may be recognized by a user via his tactile sensation or kinesthetic sensation. According to an embodiment, the haptic module 279 may include, for example, a motor, a piezoelectric element, or an electric stimulator.

The camera module 280 may capture a still image or moving images. According to an embodiment, the camera module 280 may include one or more lenses, image sensors, image signal processors, or flashes.

The power management module 288 may manage power supplied to the electronic device 201. According to an embodiment, the power management module 288 may be implemented as at least part of, for example, a power management integrated circuit (PMIC).

The battery 289 may supply power to at least one component of the electronic device 201. According to an embodiment, the battery 289 may include, for example, a primary cell which is not rechargeable, a secondary cell which is rechargeable, or a fuel cell.

The communication module 290 may support establishing a direct (e.g., wired) communication channel or a wireless communication channel between the electronic device 201 and the external electronic device (e.g., the electronic device 202, the electronic device 204, or the server 208) and performing communication via the established communication channel. The communication module 290 may include one or more communication processors that are operable independently from the processor 220 (e.g., the application processor (AP)) and supports a direct (e.g., wired) communication or a wireless communication. According to an embodiment, the communication module 290 may include a wireless communication module 292 (e.g., a cellular communication module, a short-range wireless communication module, or a global navigation satellite system (GNSS) communication module) or a wired communication module 294 (e.g., a local area network (LAN) communication module or a power line communication (PLC) module). A corresponding one of these communication modules may communicate with the external electronic device via the first network 298 (e.g., a short-range communication network, such as Bluetooth^{™}, wireless-fidelity (Wi-Fi) direct, or infrared data association (IrDA)) or the second network 299 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 292 may identify and authenticate the electronic device 201 in a communication network, such as the first network 298 or the second network 299, using subscriber information (e.g., international mobile subscriber identity (IMSI)) stored in the subscriber identification module 296.

The wireless communication module 292 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 292 may support a high-frequency band (e.g., the mmWave band) to achieve, e.g., a high data transmission rate. The wireless communication module 292 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 292 may support various requirements specified in the electronic device 201, an external electronic device (e.g., the electronic device 204), or a network system (e.g., the second network 299). According to an embodiment, the wireless communication module 292 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 297 may transmit or receive a signal or power to or from the outside (e.g., the external electronic device) of the electronic device 201. According to an embodiment, the antenna module 297 may include an antenna including a radiating element including a conductive material or a conductive pattern formed in or on a substrate (e.g., a printed circuit board (PCB)). According to an embodiment, the antenna module 297 may include a plurality of antennas (e.g., array antennas). In such a case, at least one antenna appropriate for a communication scheme used in the communication network, such as the first network 298 or the second network 299, may be selected, for example, by the communication module 290 (e.g., the wireless communication module 292) from the plurality of antennas. The signal or the power may then be transmitted or received between the communication module 290 and the external electronic device via the selected at least one antenna. According to an embodiment, another component (e.g., a radio frequency integrated circuit (RFIC)) other than the radiating element may be additionally formed as part of the antenna module 297.

According to various embodiments, the antenna module 297 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 201 and the external electronic device 204 via the server 208 coupled with the second network 299. Each of the electronic devices 202 or 204 may be a device of a same type as, or a different type, from the electronic device 201. According to an embodiment, all or some of operations to be executed at the electronic device 201 may be executed at one or more of the external electronic devices 202, 204, or 208. For example, if the electronic device 201 should perform a function or a service automatically, or in response to a request from a user or another device, the electronic device 201, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 201. The electronic device 201 may provide the outcome, with or without further processing of the outcome, as at least part of a reply to the request. To that end, a cloud computing, distributed computing, mobile edge computing (MEC), or client-server computing technology may be used, for example. The electronic device 201 may provide ultra low-latency services using, e.g., distributed computing or mobile edge computing. In an embodiment, the external electronic device 204 may include an internet-of-things (IoT) device. The server 208 may be an intelligent server using machine learning and/or a neural network. According to an embodiment, the external electronic device 204 or the server 208 may be included in the second network 299. The electronic device 201 may be applied to intelligent services (e.g., smart home, smart city, smart car, or healthcare) based on 5G communication technology or IoT-related technology.

An electronic device according to an example embodiment may include: a housing including a receiving space therein, a plurality of electrodes including an outer electrode forming at least a portion of a rear surface of the housing and an inner electrode electrically connected with the outer electrode and located in the receiving space, a wireless charging module comprising wireless charging circuitry disposed in the receiving space facing the inner electrode and having an opening formed therein, and an optical sensor module including at least one optical sensor disposed in the opening of the wireless charging module and including a sensor substrate to which the wireless charging module is coupled. The sensor substrate may be electrically connected with the plurality of electrodes by a conductive member comprising a conductive material extending from one region of the inner electrode to one side of the sensor substrate, and the wireless charging module may include a first pad configured to contact the conductive member, a second pad spaced apart from the first pad and configured to contact the sensor substrate, and a wiring connecting the first pad and the second pad.

In various example embodiments, the wireless charging module may further include a base having the opening formed in a central portion thereof, and the first pad and the second pad may extend from the base toward the opening.

In various example embodiments, the second pad may be physically and electrically connected to the sensor substrate.

In various example embodiments, a plurality of electrical signal paths may be formed between the plurality of electrodes and the sensor substrate, and the plurality of electrical signal paths may include a first electrical signal path extending from the outer electrode to the sensor substrate via the inner electrode and the conductive member and a second electrical signal path extending from the outer electrode to the sensor substrate via the inner electrode, the conductive member, the first pad, the wiring, and the second pad.

In various example embodiments, when one surface of the sensor substrate is viewed from above, the first pad may be spaced apart from the sensor substrate at a specified interval, and the second pad may at least partially overlap the sensor substrate.

In various example embodiments, the second pad may be coupled to the sensor substrate through soldering.

In various example embodiments, the sensor substrate may include a plurality of recesses formed in an edge portion thereof, and at least some of the plurality of recesses may at least partially overlap the first pad and may have the conductive member disposed therein.

In various example embodiments, the outer electrode may include a first outer electrode and a second outer electrode separated from each other. The inner electrode may include a first inner electrode and a second inner electrode separated from each other. The first inner electrode may be electrically connected with the first outer electrode, and the second inner electrode may be electrically connected with the second outer electrode.

In various example embodiments, the first pad may include a first sub-pad facing the first inner electrode and a second sub-pad facing the second inner electrode, and the plurality of recesses may include a first recess at least partially overlapping the first sub-pad and a second recess at least partially overlapping the second sub-pad.

In various example embodiments, the conductive member accommodated in the first recess may be configured to contact the first sub-pad, the first inner electrode, and a partial region of the side surface of the sensor substrate that forms the first recess, and the conductive member accommodated in the second recess may be configured to contact the second sub-pad, the second inner electrode, and a partial region of the side surface of the sensor substrate that forms the second recess.

In various example embodiments, the second pad may include a third sub-pad electrically connected with the first sub-pad and a fourth sub-pad electrically connected with the second sub-pad, and the wiring may include a first wire connecting the first sub-pad and the third sub-pad and a second wire connecting the second sub-pad and the fourth sub-pad.

In various example embodiments, the wireless charging module may further include a charging coil pattern formed on a partial region of the base and spaced apart from the wiring.

In various example embodiments, the charging coil pattern may include a spiral shape and may be spaced at a specified interval apart from an inner peripheral portion of the base that surrounds the opening, and the wiring may be formed between the inner peripheral portion and the charging coil pattern.

In various example embodiments, the wireless charging module may further include a third pad to which the charging coil pattern is electrically connected, and the third pad may be electrically isolated from the first pad and the second pad and may be physically and electrically connected to the sensor substrate.

In various example embodiments, the sensor substrate may include a first surface partially facing the inner electrode, a second surface facing away from the first surface, and a side surface connecting the first surface and the second surface. A first extension extending in a shape corresponding to the plurality of recesses may be disposed on the first surface of the sensor substrate. The first extension may be configured to contact the conductive member disposed in at least some of the plurality of recesses.

In various example embodiments, the optical sensor module may further include a light emitting part, a light receiving part, and a second extension disposed on the first surface of the sensor substrate. The light receiving part may surround a peripheral region of the light emitting part, and the second extension may be disposed between the light emitting part and the light receiving part.

In various example embodiments, the housing may include a front plate, a back plate, and a frame surrounding a space between the front plate and the back plate, and the plurality of electrodes may be disposed on a partial region of the back plate such that the outer electrode at least partially contacts a user's body.

In various example embodiments, the back plate may include a rear case coupled to the frame and a cover coupled to the rear case, and the optical sensor module and the wireless charging module may be located in a space between the rear case and the cover and may be at least partially attached to the cover.

In various example embodiments, the cover may include an inner surface facing the optical sensor module or the wireless charging module and an outer surface facing away from the inner surface. The plurality of electrodes may be configured such that the inner electrode is disposed on a partial region of the inner surface of the cover and the outer electrode extends from the inner electrode to the outer surface of the cover.

In various example embodiments, the plurality of electrodes may include a conductive layer deposited on at least a partial region of the inner surface and the outer surface of the cover.

The electronic device according to various embodiments of the disclosure may be one of various types of electronic devices. The electronic devices may include, for example, a portable communication device (e.g., a smartphone), a computer device, a portable multimedia device, a portable medical device, a camera, a wearable device, a home appliance, or the like. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), the element may be coupled with the other element directly (e.g., wiredly), wirelessly, or via a third element.

As used in connection with various embodiments of the disclosure, the term "module" may include a unit implemented in hardware, software, or firmware, or any combination thereof, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 240) including one or more instructions that are stored in a storage medium (e.g., internal memory 236 or external memory 238) that is readable by a machine (e.g., the electronic device 100 or 201). For example, a processor(e.g., the processor 220) of the machine (e.g., the electronic device 100 or 201) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Wherein, the "non-transitory" storage medium is a tangible device, and may not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

While the disclosure has been illustrated and described with reference to various example embodiments, it will be understood that the various embodiments are intended to be illustrative, not limiting. It will be further understood by those skilled in the art that various changes in form and detail may be made without departing from the invention as defined in the claims. It will also be understood that any of the embodiment(s) described herein may be used in conjunction with any other embodiment(s) described herein.

## Claims

1. An electronic device comprising:
a housing (110);
a plurality of electrodes (170) including an outer electrode defining at least a portion of a rear surface (110B) of the housing (110) and an inner electrode (174, 176), the inner electrode (174, 176) electrically connected with the outer electrode (173, 175) and located in the housing (110);
a wireless charging module (150) disposed in the housing (110) facing the inner electrode (174, 176), the wireless charging module (150) including an opening (156) formed therein; and
an optical sensor module (160) comprising at least one optical sensor disposed in the opening (156) of the wireless charging module (150), the optical sensor module (160) including a sensor substrate (161) to which the wireless charging module (150) is coupled; and
a conductive member (CR) comprising a conductive material extending from one region of the inner electrode (174, 176) to one side of the sensor substrate (161) directly electrically connecting the sensor substrate (161) and the inner electrode (174, 176), and
wherein the wireless charging module (150) includes:
a first pad (152) directly contacting the conductive member (CR) to be electrically connected to the conductive member (CR),
a second pad (153) spaced apart from the first pad (152) and directly contacting the sensor substrate (161) to be electrically connected to the sensor substrate (161), and
wiring (155) electrically connecting the first pad (152) and the second pad (153).

2. The electronic device of claim 1, wherein the wireless charging module (150) further includes a base part (151) having the opening (156) formed in a central portion thereof, and
wherein the first pad (152) and the second pad (153) extend from the base part (151) toward the opening (156).

3. The electronic device of claim 1, wherein the second pad (153) is physically and electrically connected to the sensor substrate (161).

4. The electronic device of claim 1, wherein a plurality of electrical signal paths (P1. P2) are formed between the plurality of electrodes (170) and the sensor substrate (161), and
wherein the plurality of electrical signal paths (P1, P2) include:
a first electrical signal path (P1) extending from the outer electrode (173, 175) to the sensor substrate (161) via the inner electrode (174, 176) and the conductive member (CR); and
a second electrical signal path (P2) extending from the outer electrode (173, 175) to the sensor substrate (161) via the inner electrode (174, 176), the conductive member (CR), the first pad (152), the wiring (155), and the second pad (153).

5. The electronic device of claim 1, wherein when one surface (161a, 161b) of the sensor substrate (161) is viewed from above, the first pad (152) is spaced apart from the sensor substrate (161) at a specified interval, and the second pad (153) at least partially overlaps the sensor substrate (161).

6. The electronic device of claim 1, wherein the second pad (153) is coupled to the sensor substrate (161) through soldering.

7. The electronic device of claim 1, wherein the sensor substrate (161) includes a plurality of recesses (166a, 166b, 166c) formed in an edge portion thereof, and
wherein at least some of the plurality of recesses (166a, 166b) at least partially overlap the first pad (152) and include the conductive member (CR) disposed therein.

8. The electronic device of claim 7, wherein the outer electrode (173, 175) includes a first outer electrode (173) and a second outer electrode (175) separated from each other,
wherein the inner electrode (174, 176) includes a first inner electrode (174) and a second inner electrode (176) separated from each other, and
wherein the first inner electrode (174) is electrically connected with the first outer electrode (173), and the second inner electrode (176) is electrically connected with the second outer electrode (175).

9. The electronic device of claim 8, wherein the first pad (152) includes a first sub-pad (152a) facing the first inner electrode (174) and a second sub-pad (152b) facing the second inner electrode (176), and
wherein the plurality of recesses (166a, 166b) include a first recess (166a) at least partially overlapping the first sub-pad (152a) and a second recess (166b) at least partially overlapping the second sub-pad (152b).

10. The electronic device of claim 9, wherein the conductive member (CR) accommodated in the first recess (166a) is configured to contact the first sub-pad (152a), the first inner electrode (174), and a partial region of the side surface (161c) of the sensor substrate (161) surrounding the first recess (166a), and
wherein the conductive member (CR) accommodated in the second recess (166b) is configured to contact the second sub-pad (152b), the second inner electrode (176), and a partial region of the side surface (161c) of the sensor substrate (161) surrounding the second recess (166b).

11. The electronic device of claim 9, wherein the second pad (153) includes a third sub-pad (153a) electrically connected with the first sub-pad (152a) and a fourth sub-pad (153b) electrically connected with the second sub-pad (152b), and
wherein the wiring (155) includes a first wire (155a) connecting the first sub-pad (152a) and the third sub-pad (153a) and a second wire (155b) connecting the second sub-pad (152b) and the fourth sub-pad (153b).

12. The electronic device of claim 2, wherein the wireless charging module (150) further includes a charging coil pattern (158) formed on a partial region of the base part (151) spaced apart from the wiring (155).

13. The electronic device of claim 12, wherein the charging coil pattern (158) comprises a spiral shape spaced at a specified interval apart from an inner peripheral portion (1511) of the base part (151) to surround the opening (156), and
wherein the wiring (155) is formed between the inner peripheral portion (1511) and the charging coil pattern (158).

14. The electronic device of claim 12, wherein the wireless charging module (150) further includes a third pad (154) to which the charging coil pattern (158) is electrically connected, and
wherein the third pad (154) is electrically isolated from the first pad (152) and the second pad (153) and physically and electrically connected to the sensor substrate (161).

15. The electronic device of claim 7, wherein the sensor substrate (161) includes a first surface (161a) partially facing the inner electrode (174, 176), a second surface (161b) facing away from the first surface (161a), and a side surface (161c) connecting the first surface (161a) and the second surface (161b),
wherein a first extension (164) extending in a shape corresponding to the plurality of recesses (166a, 166b, 166c) is disposed on the first surface (161a) of the sensor substrate (161), and
wherein the first extension (164) is configured to contact the conductive member disposed in at least some of the plurality of recesses (166a, 166b, 166c).

## Patentansprüche

1. Elektronische Vorrichtung, umfassend:
ein Gehäuse (110);
mehrere Elektroden (170), die eine äußere Elektrode, die zumindest einen Abschnitt einer Rückfläche (110B) des Gehäuses (110) definiert, und eine innere Elektrode (174, 176) umfassen, wobei die innere Elektrode (174, 176) elektrisch mit der äußeren Elektrode (173, 175) verbunden ist und sich im Gehäuse (110) befindet;
ein drahtloses Lademodul (150), das in dem Gehäuse (110) gegenüber der inneren Elektrode (174, 176) angeordnet ist, wobei das drahtlose Lademodul (150) eine darin ausgebildete Öffnung (156) umfasst; und
ein optisches Sensormodul (160), das mindestens einen optischen Sensor umfasst, der in der Öffnung (156) des drahtlosen Lademoduls (150) angeordnet ist, wobei das optische Sensormodul (160) ein Sensorsubstrat (161) umfasst, mit dem das drahtlose Lademodul (150) gekoppelt ist; und
ein leitfähiges Element (CR), das ein leitfähiges Material umfasst, das sich von einem Bereich der inneren Elektrode (174, 176) zu einer Seite des Sensorsubstrats (161) erstreckt und das Sensorsubstrat (161) und die innere Elektrode (174, 176) direkt elektrisch verbindet, und
wobei das drahtlose Lademodul (150) Folgendes umfasst:
ein erstes Pad (152), die das leitfähige Element (CR) direkt berührt, um mit dem leitfähigen Element (CR) elektrisch verbunden zu sein,
ein zweites Pad (153), die von dem ersten Pad (152) beabstandet ist und das Sensorsubstrat (161) direkt berührt, um mit dem Sensorsubstrat (161) elektrisch verbunden zu sein, und
eine Verdrahtung (155), die das erste Pad (152) und das zweite Pad (153) elektrisch verbindet.

2. Elektronische Vorrichtung nach Anspruch 1, wobei das drahtlose Lademodul (150) ferner einen Basisteil (151) umfasst, in dessen mittlerem Abschnitt die Öffnung (156) ausgebildet ist, und
wobei sich das erste Pad (152) und das zweite Pad (153) vom Basisteil (151) in Richtung der Öffnung (156) erstrecken.

3. Elektronische Vorrichtung nach Anspruch 1, wobei das zweite Pad (153) physikalisch und elektrisch mit dem Sensorsubstrat (161) verbunden ist.

4. Elektronische Vorrichtung nach Anspruch 1, wobei mehrere elektrische Signalpfade (P1, P2) zwischen den mehreren Elektroden (170) und dem Sensorsubstrat (161) ausgebildet sind, und
wobei die mehreren elektrischen Signalpfade (P1, P2) Folgendes umfassen:
einen ersten elektrischen Signalpfad (P1), der sich von der äußeren Elektrode (173, 175) über die innere Elektrode (174, 176) und das leitfähige Element (CR) zum Sensorsubstrat (161) erstreckt; und
einen zweiten elektrischen Signalpfad (P2), der sich von der äußeren Elektrode (173, 175) über die innere Elektrode (174, 176), das leitfähige Element (CR), das erste Pad (152), die Verdrahtung (155) und das zweite Pad (153) zum Sensorsubstrat (161) erstreckt.

5. Elektronische Vorrichtung nach Anspruch 1, wobei, wenn eine Oberfläche (161a, 161b) des Sensorsubstrats (161) von oben betrachtet wird, das erste Pad (152) in einem bestimmten Abstand vom Sensorsubstrat (161) beabstandet ist und das zweite Pad (153) das Sensorsubstrat (161) zumindest teilweise überlappt.

6. Elektronische Vorrichtung nach Anspruch 1, wobei das zweite Pad (153) durch Löten mit dem Sensorsubstrat (161) gekoppelt ist.

7. Elektronische Vorrichtung nach Anspruch 1, wobei das Sensorsubstrat (161) mehrere Aussparungen (166a, 166b, 166c) umfasst, die in einem Randabschnitt desselben ausgebildet sind, und
wobei zumindest einige der mehreren Aussparungen (166a, 166b) das erste Pad (152) zumindest teilweise überlappen und das darin angeordnete leitfähige Element (CR) umfassen.

8. Elektronische Vorrichtung nach Anspruch 7, wobei die äußere Elektrode (173, 175) eine erste äußere Elektrode (173) und eine zweite äußere Elektrode (175) umfasst, die voneinander getrennt sind,
wobei die innere Elektrode (174, 176) eine erste innere Elektrode (174) und eine zweite innere Elektrode (176) umfasst, die voneinander getrennt sind, und
wobei die erste innere Elektrode (174) mit der ersten äußeren Elektrode (173) und die zweite innere Elektrode (176) mit der zweiten äußeren Elektrode (175) elektrisch verbunden ist.

9. Elektronische Vorrichtung nach Anspruch 8, wobei das erste Pad (152) ein der ersten inneren Elektrode (174) zugewandtes erstes Unterpad (152a) und ein der zweiten inneren Elektrode (176) zugewandtes zweites Unterpad (152b) umfasst, und
wobei die mehreren Aussparungen (166a, 166b) eine erste Aussparung (166a), die das erste Unterpad (152a) zumindest teilweise überlappt, und eine zweite Aussparung (166b) umfassen, die das zweite Unterpad (152b) zumindest teilweise überlappt.

10. Elektronische Vorrichtung nach Anspruch 9, wobei das in der ersten Aussparung (166a) untergebrachte leitfähige Element (CR) so konfiguriert ist, dass es das erste Unterpad (152a), die erste innere Elektrode (174) und einen die erste Aussparung (166a) umgebenden Teilbereich der Seitenfläche (161c) des Sensorsubstrats (161) berührt, und
wobei das in der zweiten Aussparung (166b) untergebrachte leitfähige Element (CR) so konfiguriert ist, dass es das zweite Unterpad (152b), die zweite innere Elektrode (176) und einen die zweite Aussparung (166b) umgebenden Teilbereich der Seitenfläche (161c) des Sensorsubstrats (161) berührt.

11. Elektronische Vorrichtung nach Anspruch 9, wobei das zweite Pad (153) ein drittes Unterpad (153a), das elektrisch mit dem ersten Unterpad (152a) verbunden ist, und ein viertes Unterpad (153b) umfasst, das elektrisch mit dem zweiten Unterpad (152b) verbunden ist, und
wobei die Verdrahtung (155) einen ersten Draht (155a), der das erste Unterpad (152a) und das dritte Unterpad (153a) verbindet, und einen zweiten Draht (155b) umfasst, der das zweite Unterpad (152b) und das vierte Unterpad (153b) verbindet.

12. Elektronische Vorrichtung nach Anspruch 2, wobei das drahtlose Lademodul (150) ferner ein Ladespulenmuster (158) umfasst, das auf einem Teilbereich des Basisteils (151) ausgebildet ist, der von der Verdrahtung (155) beabstandet ist.

13. Elektronische Vorrichtung nach Anspruch 12, wobei das Ladespulenmuster (158) eine Spiralform umfasst, die in einem bestimmten Abstand von einem inneren Umfangsabschnitt (1511) des Basisteils (151) beabstandet ist, um die Öffnung (156) zu umgeben, und
wobei die Verdrahtung (155) zwischen dem inneren Umfangsabschnitt (1511) und dem Ladespulenmuster (158) ausgebildet ist.

14. Elektronische Vorrichtung nach Anspruch 12, wobei das drahtlose Lademodul (150) ferner ein drittes Pad (154) umfasst, mit dem das Ladespulenmuster (158) elektrisch verbunden ist, und
wobei das dritte Pad (154) elektrisch von dem ersten Pad (152) und dem zweiten Pad (153) isoliert und physikalisch sowie elektrisch mit dem Sensorsubstrat (161) verbunden ist.

15. Elektronische Vorrichtung nach Anspruch 7, wobei das Sensorsubstrat (161) eine erste Oberfläche (161a), die teilweise der inneren Elektrode (174, 176) zugewandt ist, eine zweite Oberfläche (161b), die von der ersten Oberfläche (161a) abgewandt ist, und eine Seitenfläche (161c) umfasst, die die erste Oberfläche (161a) und die zweite Oberfläche (161b) verbindet,
wobei eine erste Verlängerung (164), die sich in einer Form erstreckt, die den mehreren Aussparungen (166a, 166b, 166c) entspricht, an der ersten Oberfläche (161a) des Sensorsubstrats (161) angeordnet ist, und
wobei die erste Verlängerung (164) dazu konfiguriert ist, das in mindestens einigen der mehreren Aussparungen (166a, 166b, 166c) angeordnete leitfähige Element zu berühren.

## Revendications

1. Dispositif électronique comprenant :
un boîtier (110) ;
une pluralité d'électrodes (170) comportant une électrode externe définissant au moins une partie d'une surface arrière (110B) du boîtier (110) et une électrode interne (174, 176), l'électrode interne (174, 176) étant électriquement connectée avec l'électrode externe (173, 175) et située dans le boîtier (110) ;
un module de charge sans fil (150) disposé dans le boîtier (110) faisant face à l'électrode interne (174, 176), le module de charge sans fil (150) comportnant une ouverture (156) formée dans celui-ci ; et
un module de capteur optique (160) comprenant au moins un capteur optique disposé dans l'ouverture (156) du module de charge sans fil (150), le module de capteur optique (160) comportant un substrat de capteur (161) auquel le module de charge sans fil (150) est couplé ; et
un élément conducteur (CR) comprenant un matériau conducteur s'étendant depuis une région de l'électrode interne (174, 176) jusqu'à un côté du substrat de capteur (161) connectant électriquement directement le substrat de capteur (161) et l'électrode interne (174, 176), et
dans lequel le module de charge sans fil (150) comporte :
un premier plot (152) venant directement en contact avec l'élément conducteur (CR) pour être électriquement connecté à l'élément conducteur (CR),
un deuxième plot (153) espacé du premier plot (152) et venant directement en contact avec le substrat de capteur (161) pour être électriquement connecté au substrat de capteur (161), et
un câblage (155) connectant électriquement le premier plot (152) et le deuxième plot (153).

2. Dispositif électronique selon la revendication 1, dans lequel le module de charge sans fil (150) comporte en outre une partie de base (151) ayant l'ouverture (156) formée dans une partie centrale de celle-ci, et
dans lequel le premier plot (152) et le deuxième plot (153) s'étendent depuis la partie de base (151) vers l'ouverture (156).

3. Dispositif électronique selon la revendication 1, dans lequel le deuxième plot (153) est physiquement et électriquement connecté au substrat de capteur (161).

4. Dispositif électronique selon la revendication 1, dans lequel une pluralité de trajets de signal électrique (P1, P2) sont formés entre la pluralité d'électrodes (170) et le substrat de capteur (161), et
dans lequel la pluralité de trajets de signal électrique (P1, P2) comportent :
un premier trajet de signal électrique (P1) s'étendant depuis l'électrode externe (173, 175) jusqu'au substrat de capteur (161) via l'électrode interne (174, 176) et l'élément conducteur (CR) ; et
un second trajet de signal électrique (P2) s'étendant depuis l'électrode externe (173, 175) jusqu'au substrat de capteur (161) via l'électrode interne (174, 176), l'élément conducteur (CR), le premier plot (152), le câblage (155), et le deuxième plot (153).

5. Dispositif électronique selon la revendication 1, dans lequel lorsque une surface (161a, 161b) du substrat de capteur (161) est vue depuis le dessus, le premier plot (152) est espacé du substrat de capteur (161) à un intervalle spécifié, et le deuxième plot (153) chevauche au moins partiellement le substrat de capteur (161).

6. Dispositif électronique selon la revendication 1, dans lequel le deuxième plot (153) est couplé au substrat de capteur (161) par soudage.

7. Dispositif électronique selon la revendication 1, dans lequel le substrat de capteur (161) comporte une pluralité d'évidements (166a, 166b, 166c) formés dans une partie de bord de celui-ci, et
dans lequel au moins certains de la pluralité d'évidements (166a, 166b) chevauchent au moins partiellement le premier plot (152) et comportent l'élément conducteur (CR) disposé dans ceux-ci.

8. Dispositif électronique selon la revendication 7, dans lequel l'électrode externe (173, 175) comporte une première électrode externe (173) et une seconde électrode externe (175) séparées l'une de l'autre,
dans lequel l'électrode interne (174, 176) comporte une première électrode interne (174) et une seconde électrode interne (176) séparées l'une de l'autre, et
dans lequel la première électrode interne (174) est électriquement connectée avec la première électrode externe (173), et la seconde électrode interne (176) est électriquement connectée avec la seconde électrode externe (175).

9. Dispositif électronique selon la revendication 8, dans lequel le premier plot (152) comporte un premier sous-plot (152a) faisant face à la première électrode interne (174) et un deuxième sous-plot (152b) faisant face à la seconde électrode interne (176), et
dans lequel la pluralité d'évidements (166a, 166b) comprennent un premier évidement (166a) chevauchant au moins partiellement le premier sous-plot (152a) et un second évidement (166b) chevauchant au moins partiellement le deuxième sous-plot (152b).

10. Dispositif électronique selon la revendication 9, dans lequel l'élément conducteur (CR) logé dans le premier évidement (166a) est configuré pour venir en contact avec le premier sous-plot (152a), la première électrode interne (174), et une région partielle de la surface latérale (161c) du substrat de capteur (161) entourant le premier évidement (166a), et
dans lequel l'élément conducteur (CR) logé dans le second évidement (166b) est configuré pour venir en contact avec le deuxième sous-plot (152b), la seconde électrode interne (176), et une région partielle de la surface latérale (161c) du substrat de capteur (161) entourant le second évidement (166b).

11. Dispositif électronique selon la revendication 9, dans lequel le deuxième plot (153) comporte un troisième sous-plot (153a) électriquement connecté avec le premier sous-plot (152a) et un quatrième sous-plot (153b) électriquement connecté avec le deuxième sous-plot (152b), et
dans lequel le câblage (155) comporte un premier fil (155a) connectant le premier sous-plot (152a) et le troisième sous-plot (153a) et un second fil (155b) connectant le deuxième sous-plot (152b) et le quatrième sous-plot (153b).

12. Dispositif électronique selon la revendication 2, dans lequel le module de charge sans fil (150) comprend en outre un motif de bobine de charge (158) formé sur une région partielle de la partie de base (151) espacée du câblage (155).

13. Dispositif électronique selon la revendication 12, dans lequel le motif de bobine de charge (158) comprend une forme en spirale espacée à un intervalle spécifié d'une partie périphérique interne (1511) de la partie de base (151) pour entourer l'ouverture (156), et
dans lequel le câblage (155) est formé entre la partie périphérique interne (1511) et le motif de bobine de charge (158).

14. Dispositif électronique selon la revendication 12, dans lequel le module de charge sans fil (150) comporte en outre un troisième plot (154) auquel le motif de bobine de charge (158) est électriquement connecté, et
dans lequel le troisième plot (154) est électriquement isolé du premier plot (152) et du deuxième plot (153) et physiquement et électriquement connecté au substrat de capteur (161).

15. Dispositif électronique selon la revendication 7, dans lequel le substrat de capteur (161) comporte une première surface (161a) faisant partiellement face à l'électrode interne (174, 176), une seconde surface (161b) faisant face à l'opposé de la première surface (161a), et une surface latérale (161c) connectant la première surface (161a) et la seconde surface (161b),
dans lequel une première extension (164) s'étendant dans une forme correspondant à la pluralité d'évidements (166a, 166b, 166c) est disposée sur la première surface (161a) du substrat de capteur (161), et
dans lequel la première extension (164) est configurée pour venir en contact avec l'élément conducteur disposé dans au moins certains de la pluralité d'évidements (166a, 166b, 166c).
